# EUROPEAN PATENT SPECIFICATION

(11) **EP 4 205 716 B1**
(45) Date of publication and mention of the grant of the patent: **22.10.2025**
(21) Application number: 21875813.4
(22) Date of filing: 30.09.2021
(51) Int. Cl.: A61F 13/494, A61F 13/51, A61F 13/514

(54) **UNDERPANTS-TYPE ABSORBENT ARTICLE**
SAUGFÄHIGER ARTIKEL VOM UNTERHOSEN-TYP
ARTICLE ABSORBANT DE TYPE CULOTTE

(30) Priority: 30.09.2020 CN 202011059957; 30.09.2020 CN 202011057355
(43) Date of publication of application: 05.07.2023
(73) Proprietor: Unicharm Corporation, Ehime 799-0111 (JP)
(72) Inventor: KAWAKAMI, Yusuke, Kanonji-shi, Kagawa 769-1602 (JP); NAGAI, Takahito, Kanonji-shi, Kagawa 769-1602 (JP); TANAKA, Suguru, Kanonji-shi, Kagawa 769-1602 (JP); WANG, Yinhua, Shanghai 201700 (CN); ZHENG, Lingshuang, Shanghai 201700 (CN)
(74) Representative: Dolleymores
(86) International application number: PCT/JP2021/036271
(87) International publication number: WO 2022/071517

(56) References cited:
- JP-A- 2001 327 534
- JP-A- 2003 220 091
- JP-A- 2004 344 529
- JP-A- 2004 344 529
- JP-A- 2015 058 034
- JP-A- 2015 058 034
- JP-A- 2016 087 286
- US-A1- 2011 046 590

## Description

### FIELD

The present invention relates to an underpants-shaped absorbent article.

### BACKGROUND

Conventionally, in an absorbent article such as an underpants-shaped disposable diaper, there has been known a technique for increasing water absorbency by using a hydrophilic sheet for a sheet member constituting the absorbent article. For example, Patent Literature 1 discloses a disposable diaper in which a sheet member such as a nonwoven fabric containing hydrophilic fibers is used in a skin-side sheet 5 located on the skin side of a wearer and an outer-layer sheet 4 located on the non-skin side.

Further, there has been known an underpants-shaped diaper using a hydrophilic nonwoven fabric whose hydrophilicity is enhanced by including a hydrophilic oil agent. For example, Patent Literature 1 discloses a technique related to a disposable diaper in which a hydrophilic nonwoven fabric is used as an exterior sheet 4 that constitutes an exterior body 3 of a diaper 1, the hydrophilic nonwoven fabric having hydrophilized fibers which are obtained by means such as making hydrophobic synthetic fibers undergo treatment with a hydrophilizing agent.

### [CITATION LIST]

### [PATENT LITERATURE]

Patent Literature 1: Japanese Patent Application Publication No. 2017-113186

### SUMMARY

### [TECHNICAL PROBLEM]

A problem to be solved by the present invention is at least one of a first problem and a second problem below.

The first problem is as follow. In a diaper using such a hydrophilic sheet member, moisture such as sweat can be likely to be absorbed from the wearer's skin while the diaper is put on. However, in the case where moisture is absorbed by the hydrophilic sheet, there is a case where a wet hydrophilic sheet containing moisture comes into contact with the wearer's skin, causing problems such as rashes to the wearer's skin or making the wearer to feel discomfort. In particular, in the crotch portion of the absorbent article, when the wearer moves the legs, the wet sheet member is likely to come into contact with the leg-circumferential region, and there is a risk of causing discomfort due to stickiness in the crotch portion.

The present invention was achieved in light of conventional problems such as that described above and an aspect of the present invention is that, in an underpants-shaped absorbent article including a hydrophilic sheet and having enhanced water absorbency, it is to suppress discomfort in a crotch portion during usage.

A second problem is as follow. In a diaper using such a hydrophilic sheet member, moisture such as sweat can be likely to be absorbed from the wearer's skin while the diaper is put on. However, in the manufacturing step, when forming the diaper into an underpants shape, there is a case where in side joining portions in which the front panel (front waist portion) and the back panel (back waist portion) are joined, the joining strength of the side joining portions is decreased due to the effect of a hydrophilic oil agent. For example, in the case where the side joining portions are formed by joining means such as thermal welding or ultrasonic welding, there is a risk that a sufficient joining strength cannot be obtained when a coating of the hydrophilic oil agent is interposed between the facing surfaces of the front waist portion and the back waist portion. In such a case, when the wearer puts on or takes off the diaper, there is a risk that, due to a load applied to the lower end portions of the side joining portions or the like, the side joining portions are likely to peel off, making it impossible to maintain the underpants shape.

The present invention was achieved in light of conventional problems such as that described above, and an aspect of the present invention is that, in an underpants-shaped absorbent article having a hydrophilic sheet containing a hydrophilic oil agent, it is to suppress the peeling off the lower end portions of side joining portions.

JP2004344529, JP2015058403 and US2011/046590 disclose underpants-shaped absorbent articles.

### [SOLUTION TO PROBLEM]

The present invention provides an underpants-shaped absorbent article as claimed in claim 1.

Other features are disclosed in the dependent claims.

Features of the present invention other than the above will become clear by reading the description of the present specification with reference to the accompanying drawings.

### [ADVANTAGEOUS EFFECT OF THE INVENTION]

An effect of the present invention on the first problem is that, in an underpants-shaped absorbent article including a hydrophilic sheet and having enhanced water absorbency, it is to suppress discomfort in a crotch portion during usage.

An effect of the present invention on the second problem is that, in an underpants-shaped absorbent article having a hydrophilic sheet containing a hydrophilic oil agent, it is to suppress the peeling off of the lower end portions of side joining portions.

### BRIEF DESCRIPTION OF DRAWINGS

FIG. 1 is a schematic perspective view of a diaper 1.
FIG. 2 is a plan view of the diaper 1 in an unfolded and stretched state.
FIG. 3 is a schematic cross-sectional view taken along a line A-A in FIG. 2.
FIG. 4A is a plan view of an absorbent main body 10, and FIG. 4B is a schematic cross-sectional view of the absorbent main body 10.
FIGS. 5A to 5C are explanatory diagrams illustrating the basic principle when moisture is absorbed and evaporated in the diaper 1.
FIG. 6A is an explanatory diagram illustrating an experimental method for measuring the evaporation rate.
FIG. 6B is a graph showing evaporation rate data obtained from experimental results.
FIGS. 7A and 7B are diagrams illustrating how the absorbent main body 10 fits to the crotch portion of a wearer while the diaper 1 is put on.
FIG. 8 is an explanatory diagram illustrating a method for attaching a waist elastic member 35 to a front waist portion 30 using welding portions 60.
FIG. 9 is a plan view of a diaper 2 in an unfolded and stretched state.
FIG. 10 is a schematic cross-sectional view taken along a line B-B in FIG. 9.
FIG. 11 is a plan view of a diaper 3 in an unfolded and stretched state.
FIG. 12 is a schematic cross-sectional view taken along a line C-C in FIG. 11.
FIG. 13 is a plan view of a modified example of the diaper 3 in an unfolded and stretched state.
FIG. 14 is a schematic cross-sectional view taken along a line D-D in FIG. 13.
FIG. 15 is a plan view of a diaper 4 in an unfolded and stretched state.
FIG. 16 is a schematic cross-sectional view of a side joining portion 50 that joins the front waist portion 30 and a back waist portion 40, when viewed in the vertical direction.
FIG. 17 is a schematic cross-sectional view showing a cross-sectional shape of the front waist portion 30 in the thickness direction.
FIG. 18 is a schematic cross-sectional view showing the relationship between the positions of compressed portions 31e of the front waist portion 30 and the positions of compressed portions 41e of the back waist portion 40 in the side joining portion 50.
FIG. 19A is a plan view of a diaper 5 in an unfolded and stretched state.
FIG. 19B is a schematic cross-sectional view taken along a line B-B in FIG. 19A.
FIG. 20A is a plan view of a diaper 6 in an unfolded and stretched state.
FIG. 20B is a schematic cross-sectional view taken along a line C-C in FIG. 20A.

### DESCRIPTION OF EMBODIMENTS

At least following matters will become clear with description of this specification and attached drawings.

An underpants-shaped absorbent article having a vertical direction, a lateral direction, and a front-back direction that intersect with each other, the underpants-shaped absorbent article including: a liquid-absorbent absorbent main body; a waist member in which a front waist portion and a back waist portion are annularly joined by a pair of side joining portions that is provided at two end portions in the lateral direction; and a pair of leg openings through which a wearer's legs are passed, in the waist member, at least in a part of a region that overlap the pair of side joining portions with respect to the vertical direction, a hydrophilic region being provided farthest on a non-skin side, in regions extending along the leg openings, at least in a portion that are located at a lowermost end, a hydrophobic region being provided farthest on the non-skin side, the hydrophobic region having a lower hydrophilicity than the hydrophilic region.

According to the above-described underpants-shaped absorbent article, the hydrophilic region located on the non-skin side of the waist member makes it possible to absorb moisture from the wearer's skin and to evaporate the absorbed moisture into the atmosphere. Further, the lowermost end portion of the leg opening that comes into contact with the wearer's crotch portion during usage of the absorbent article is a hydrophobic region, and this makes the wearer's crotch portion less likely to become wet. Accordingly, moisture is less likely to come into contact with the wearer's skin, and particularly, in the crotch portion, discomfort can be suppressed.

In such an underpants-shaped absorbent article, it is desirable that in an unfolded state, the front waist portion is joined to a one side of the absorbent main body in a longitudinal direction, and the back waist portion is joined to another side in the longitudinal direction, that a hydrophilic nonwoven fabric is provided farthest on the non-skin side in at least one of the front waist portion and the back waist portion, and that a hydrophobic nonwoven fabric is provided farthest on a non-skin side in the absorbent main body, the hydrophobic nonwoven fabric having a lower hydrophilicity than the hydrophilic nonwoven fabric.

According to the above-described underpants-shaped absorbent article, in an underpants-shaped absorbent article having a three-piece structure, by using a hydrophobic nonwoven fabric as a sheet member of the outermost layer of the absorbent main body that comes into contact with the wearer's crotch portion, the crotch portion is less likely to become wet during usage of the absorbent article, and the wearer can be less likely to feel discomfort.

In such an underpants-shaped absorbent article, it is desirable that in an unfolded state, the waist member is integrally constituted such that the front waist portion and the back waist portion are continuous in a longitudinal direction, that the waist member is joined to a non-skin side of the absorbent main body, that a hydrophilic nonwoven fabric is provided farthest on a non-skin side of the waist member, that a hydrophobic nonwoven fabric is provided farthest on a non-skin side of the absorbent main body, the hydrophobic nonwoven fabric having a lower hydrophilicity than the hydrophilic nonwoven fabric, and that a minimum value of a lateral width of the waist member is smaller than a minimum value of a lateral width of the absorbent main body.

According to the above-described underpants-shaped absorbent article, in an underpants-shaped absorbent article having a two-piece structure, by using the hydrophobic nonwoven fabric as a sheet member of the outermost layer of the absorbent main body that comes into contact with the wearer's crotch portion, the crotch portion is less likely to become wet during usage of the absorbent article, and the wearer can be less likely to feel discomfort.

In such an underpants-shaped absorbent article, it is desirable that in the lateral direction, a distance between an outermost end of the absorbent main body and an innermost end of the waist member is less than 1/2 of a distance between an outermost end of the waist member and the innermost end of the waist member.

According to the underpants-shaped absorbent article, the side edge portions of the absorbent main body (crotch leg-circumferential region) are prevented from excessively extending outward in the lateral direction. Accordingly, while the absorbent article is put on, the wearer can easily move the legs and be less likely to feel discomfort around the legs.

In such an underpants-shaped absorbent article, it is desirable that the leg opening has a first region and a second region on and below a lower end of the side joining portion, the first region being constituted by the waist member, the second region being constituted by the absorbent main body, that a portion of the first region farthest on the non-skin side is a hydrophilic region, and that a portion of the second region farthest on the non-skin side is a hydrophobic region.

According to the above-described underpants-shaped absorbent article, in the leg openings of the underpants-shaped absorbent article having the three-piece structure or the two-piece structure, moisture is likely to be evaporated in the first region, and moisture is less likely to be held in the second region that comes into contact with the wearer's crotch portion, making the wearer's crotch portion less likely to become wet. Accordingly, the wearer can be less likely to feel discomfort in a wide range of the crotch portion.

In such an underpants-shaped absorbent article, it is desirable that in an unfolded state, a length of a portion that extends along the second region and that is located on a front side with respect to a central position of the underpants-shaped absorbent article in the longitudinal direction is longer than a length of a portion that extends along the second region and that is located on a back side with respect to the central position.

According to the above-described underpants-shaped absorbent article, the hydrophobic region on the front side in the crotch portion of the leg opening is wide, making the wearer less likely to become wet around the legs even in the case where the wearer moves the legs forward, such as when walking or the like. Accordingly, the wearer can be less likely to feel discomfort while the diaper is put on.

In such an underpants-shaped absorbent article, it is desirable that in an unfolded state, a length of a portion that extends along the first region and that is located on a back side with respect to a central position of the underpants-shaped absorbent article in the longitudinal direction is longer than a length of a portion that extends along the first region and that is located on a front side with respect to the central position.

According to the above-described underpants-shaped absorbent article, in the leg opening, it makes wide the hydrophobic region in the back leg-circumferential region that comes into wide contact with the wearer's buttocks and to which the body pressure is likely to be applied when the wearer sits down. This makes the buttocks less likely to become wet, and the wearer can be less likely to feel discomfort.

In such an underpants-shaped absorbent article, it is desirable that the front waist portion has a front skin-side sheet and a front non-skin-side sheet, that the back waist portion has a back skin-side sheet and a back non-skin-side sheet, that the front skin-side sheet and the back skin-side sheet are formed of an integral sheet member that is continuous in the longitudinal direction in an unfolded state, that the front non-skin-side sheet and the back non-skin-side sheet are formed of different sheet members that are non-continuous in the longitudinal direction in the unfolded state, that the waist member is joined to a non-skin side of the absorbent main body, that the different sheet members that constitute the front non-skin-side sheet and the back non-skin-side sheet are hydrophilic nonwoven fabrics, and that the integral sheet member that constitutes the front skin-side sheet and the back skin-side sheet is a hydrophobic nonwoven fabric having a lower hydrophilicity than the hydrophilic nonwoven fabric.

According to the above-described underpants-shaped absorbent article, in an underpants-shaped absorbent article having a simple three-piece structure, by using the hydrophobic nonwoven fabric as a sheet member in the outermost layer that comes into contact with the wearer's crotch portion (integral sheet member), the crotch portion is less likely to become wet during usage of the absorbent article, and the wearer can be less likely to feel discomfort.

In such an underpants-shaped absorbent article, it is desirable that a minimum value of a lateral width of the waist member is larger than a minimum value of a lateral width of the absorbent main body, that the leg opening is formed of the waist member and is located below a lower end of the side joining portion, that the leg opening has a first region and a second region, the first region including the front non-skin-side sheet and the back non-skin-side sheet, the second region not including the front non-skin-side sheet and the back non-skin-side sheet, and being constituted by the front skin-side sheet and the back skin-side sheet, and that a portion of the first region farthest on the non-skin side is a hydrophilic region, and that a portion of the second region farthest on the non-skin side is a hydrophobic region.

According to the above-described underpants-shaped absorbent article, in the leg openings of the underpants-shaped absorbent article having the simple three-piece structure, moisture is likely to be evaporated in the first region, and moisture is less likely to be held in the second region that comes into contact with the wearer's crotch portion, making the wearer's crotch portion less likely to become wet. Accordingly, the wearer can be less likely to feel discomfort in a wide range of the crotch portion.

In such an underpants-shaped absorbent article, it is desirable that in the second region, a sheet member arranged on a non-skin side with respect to the absorbent main body is only one sheet of the hydrophobic nonwoven fabric.

According to the above-described underpants-shaped absorbent article, while the absorbent article is put on, the texture of the crotch portions of the leg openings against the skin is soft, and this allows the wearer to feel comfortable.

In such an underpants-shaped absorbent article, it is desirable that a minimum value of a lateral width of the waist member is smaller than a minimum value of a lateral width of the absorbent main body, that the leg opening has a first region and a second region on and below a lower end of the side joining portion, the first region being constituted by the waist member, the second region being constituted by the absorbent main body, that a portion of the first region farthest on the non-skin side is a hydrophilic region, and that a portion of the second region farthest on the non-skin side is a hydrophobic region.

According to the above-described underpants-shaped absorbent article, in the leg openings of the underpants-shaped absorbent article having the simple three-piece structure, moisture is likely to be evaporated in the first region, and moisture is less likely to be held in the second region that comes into contact with the wearer's crotch portion, making the wearer's crotch portion less likely to become wet. Accordingly, the wearer can be less likely to feel discomfort in a wide range of the crotch portion.

In such an underpants-shaped absorbent article, it is desirable that in an unfolded state, a length of a portion that extends along the second region and that is located on a front side with respect to a central position of the underpants-shaped absorbent article in the longitudinal direction is longer than a length of a portion that extends along the second region and that is located on a back side with respect to the central position.

According to the above-described underpants-shaped absorbent article, the hydrophobic region on the front side in the crotch portion of the leg opening is wide, making the wearer less likely to become wet around the legs even in the case where the wearer moves the legs forward, such as when walking or the like. Accordingly, the wearer can be less likely to feel discomfort while the diaper is put on.

In such an underpants-shaped absorbent article, it is desirable that in an unfolded state, a length of a portion that extends along the first region and that is located on a back side with respect to a central position of the underpants-shaped absorbent article in the longitudinal direction is longer than a length of a portion that extends along the first region and that is located on a front side with respect to the central position.

According to the above-described underpants-shaped absorbent article, in the leg opening, it makes wide the hydrophobic region in the back leg-circumferential region that comes into wide contact with the wearer's buttocks and to which the body pressure is likely to be applied when the wearer sits down. This makes the buttocks less likely to become wet, and the wearer can be less likely to feel discomfort.

In such an underpants-shaped absorbent article, it is desirable that a farthest-on-non-skin-side layer of each of the front waist portion and the back waist portion is formed of one sheet of hydrophilic nonwoven fabric.

According to the above-described underpants-shaped absorbent article, the non-skin-side surfaces of the front waist portion and the back waist portion are each formed of one sheet of hydrophilic nonwoven fabric, and therefore a hydrophilic region is formed in a wide range of the front waist portion and the back waist portion, making it possible to further enhance the evaporation property. Further, not providing extra sheet member makes it less likely to inhibit the absorption and evaporation of moisture, making it possible to more efficiently discharge the moisture.

In such an underpants-shaped absorbent article, it is desirable that the back waist portion has a buttocks cover below lower ends of the side joining portions, the buttocks cover having a lateral width that is narrowed from an upper side to a lower side in the vertical direction, and that an elastic member that stretches and contracts along an end edge portion of the buttocks cover is provided.

According to the above-described underpants-shaped absorbent article, the buttocks cover can make moisture such as sweat likely to evaporate in the wearer's buttocks. Further, the stretchability of the elastic member located on the buttocks cover makes the buttocks cover likely to fit to the wearer's buttocks while the absorbent article is put on, and makes the buttocks side of the leg opening less likely to turn up. Accordingly, the wearer can be less likely to feel unpleasantness or discomfort.

Further, an underpants-shaped absorbent article having a vertical direction, a lateral direction, and a front-back direction that intersect with each other, the underpants-shaped absorbent article including: a liquid-absorbent absorbent main body; and a waist member in which a front waist portion and a back waist portion are annularly joined by a pair of side joining portions that is provided at two side portions in the lateral direction, the waist member having a hydrophilic sheet portion and a hydrophobic sheet portion, the hydrophilic sheet portion containing a hydrophilic oil agent, the hydrophobic sheet portion containing a smaller amount of the hydrophilic oil agent per unit volume than the hydrophilic sheet portion, in a lower end portion of the side joining portion, a farthest-on-skin-side surface of the front waist portion and a farthest-on-skin-side surface of the back waist portion are joined facing each other, the hydrophobic sheet portion is provided in at least one of the farthest-on-skin-side surface of the front waist portion and the farthest-on-skin-side surface of the back waist portion.

According to the above-described underpants-shaped absorbent article, the hydrophobic sheet portion having a low hydrophilic oil agent content is provided in at least one of the sheet members that face each other in the thickness direction at the lower end portion of the side joining portion. Accordingly, the influence of the hydrophilic oil agent in the side joining portion is reduced compared with the case where both of the sheet members that face each other in the thickness direction are hydrophilic sheet portions. That is, the joining strength at the lower end portion of the side joining portion is less likely to decrease. This suppresses the peeling of the lower end portion of the side joining portion at the time of putting on the absorbent article, enabling to make the underpants shape likely to be maintained.

In such an underpants-shaped absorbent article, it is desirable that in the lower end portion of the side joining portion, the hydrophobic sheet portion is provided on both of the farthest-on-skin-side surface of the front waist portion and the farthest-on-skin-side surface of the back waist portion.

According to the above-described underpants-shaped absorbent article, in the lower end portion of the side joining portion, all of the sheet members that face each other in the thickness direction are the hydrophobic sheet portions, and accordingly the influence of the hydrophilic oil agent contained in the hydrophilic sheet portions is further reduced. Therefore, the joining strength of the side joining portion can be more easily prevented from being decreased.

In such an underpants-shaped absorbent article, it is desirable that the hydrophilic sheet portion is provided on both of a farthest-on-non-skin-side surface of the front waist portion and a farthest-on-non-skin-side surface of the back waist portion.

According to the above-described underpants-shaped absorbent article, the moisture absorbed from the wearer's skin by the hydrophobic sheet portion located on the skin side can be transferred to the hydrophilic sheet portion located on the non-skin side and can be easily released to the atmosphere from the non-skin-side surface of the hydrophilic sheet portion. Accordingly, moisture is less likely to come into contact with the wearer's skin, and this makes it possible to suppress the occurrence of skin problems or for the wearer to be less likely to feel discomfort.

In such an underpants-shaped absorbent article, it is desirable that the hydrophobic sheet portion is provided with a plurality of compressed portions in which the hydrophobic sheet portion is compressed from a non-skin side to a skin side.

According to the above-described underpants-shaped absorbent article, the bottom portions of the compressed portions are arranged at the positions spaced apart by a predetermined distance in the thickness direction from the hydrophilic sheet portion located on the non-skin side. A portion of the hydrophobic sheet portion in which the compressed portions are provided is less likely to be affected by the hydrophilic oil agent contained in the hydrophilic sheet portion. Accordingly, it is possible to make it easier to suppress the decrease in the joining strength of the side joining portion.

In such an underpants-shaped absorbent article, it is desirable that the underpants-shaped absorbent article has a portion where a position of the compressed portion provided in the front waist portion and a position of the compressed portion provided in the back waist portion do not overlap each other when the side joining portion is viewed in a thickness direction.

According to the above-described underpants-shaped absorbent article, since the bottom portions of the compressed portions having high stiffness are arranged at positions displaced from each other, the stiffness of the side joining portions is prevented from becoming excessively high, and the texture while the absorbent article is put on is less likely to deteriorate. Further, it suppresses welding defects occurring at the time of the formation of the side joining portion due to the overlapping of the bottom portions of the compressed portions having high stiffness, and this can make the side joining portions less likely to peel off.

In such an underpants-shaped absorbent article, it is desirable that two or more layers of the hydrophobic sheet portion are provided farthest on a skin side between a central position and a lower end position in the two side portions of the waist member in the vertical direction.

According to the above-described underpants-shaped absorbent article, since two or more layers of the hydrophobic sheet portion are provided on the skin-side surface of the waist member, the influence of the hydrophilic oil agent contained in the hydrophilic sheet portion arranged on the non-skin side is reduced in the lower end portion of the side joining portion. This can make it easier to suppress a decrease in the joining strength.

In such an underpants-shaped absorbent article, it is desirable that a hydrophobic skin surface sheet is provided on a farthest-on-skin-side surface of the waist member, and that a lower end of the skin surface sheet is positioned between the central position and the lower end position in the two side portions of the waist member in the vertical direction.

According to the above-described underpants-shaped absorbent article, even in the case where peeling occurs at the lower end portion of the side joining portion while the absorbent article is put on and the peeling propagates toward the upper side in the vertical direction, the propagation of the peeling can be stopped at the lower end position of the skin surface sheet. Accordingly, the peeling of the entire side joining portion can be suppressed, and the underpants shape of the absorbent article can be more likely to be maintained.

In such an underpants-shaped absorbent article, it is desirable that the hydrophobic sheet portion is provided farthest on the skin-side surfaces of the front waist portion and the back waist portion in an entire region of the side joining portion from an upper end to a lower end in the vertical direction.

According to the above-described underpants-shaped absorbent article, a decrease in the joining strength in the entire region of the side joining portion is likely to be suppressed, and the underpants shape of the absorbent article can be likely to be stably maintained.

In such an underpants-shaped absorbent article, it is desirable that an amount of the hydrophilic oil agent contained per unit volume in an upper end portion of the side joining portion is larger than an amount of the hydrophilic oil agent contained per unit volume in the lower end portion of the side joining portion.

According to the above-described underpants-shaped absorbent article, since the hydrophilic oil agent content is higher in the upper end portion of the side joining portion than in the lower end portion of the side joining portion, the joining strength is weakened. This makes it easier to perform an operation of tearing the side joining portion from the upper end portion side when removing the absorbent article from the wearer's body. Accordingly, the user can perform an operation of putting on and taking off the absorbent article without any stress.

In such an underpants-shaped absorbent article, it is desirable that two or more layers of the hydrophilic sheet portion are provided in an upper end portion of the side joining portion.

According to the above-described underpants-shaped absorbent article, in the upper end portion of the side joining portion, it is possible to increase the strength of the waist member. Therefore, when removing the absorbent article, even if the front waist portion and the back waist portion are pulled in the front-back direction by grabbing the upper end portion of the side joining portion, it makes less likely to tear the sheet members constituting the front waist portion and the back waist portion. Also, it makes a pulling force likely to act to peel off the side joining portions. This makes it easier to perform the operation of peeling off the side joining portions from the upper end portion side to the lower side.

### First Embodiment

The following describes an underpants-shaped absorbent article according to the present invention by way of example of an underpants-shaped disposable diaper (hereinafter, also referred to as a "diaper 1"). However, the underpants-shaped absorbent article according to the present invention includes an underpants-shaped napkin, and other types of underpants-shaped absorbent article.

### Configuration of Diaper 1

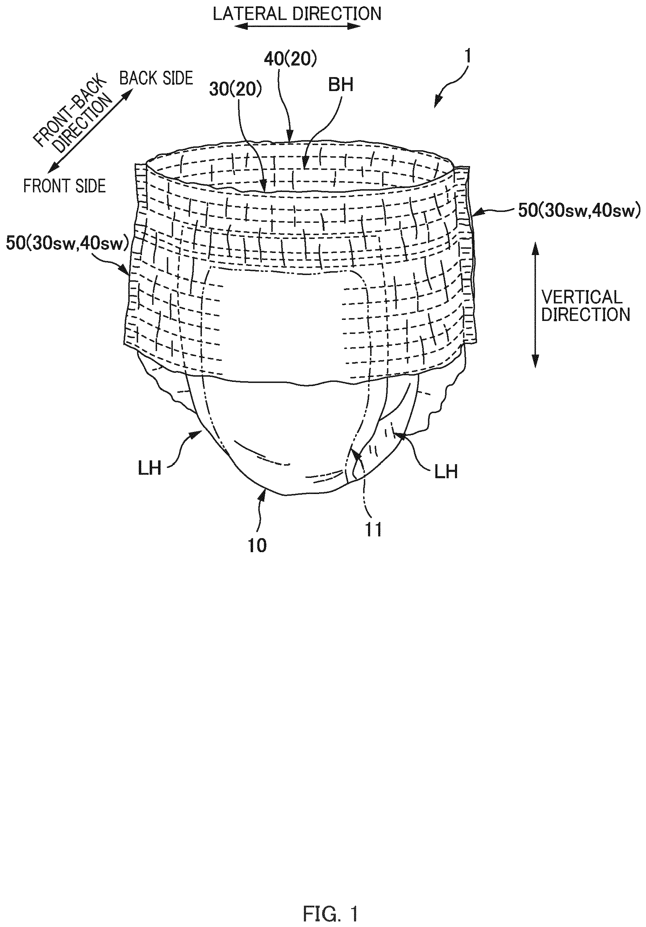

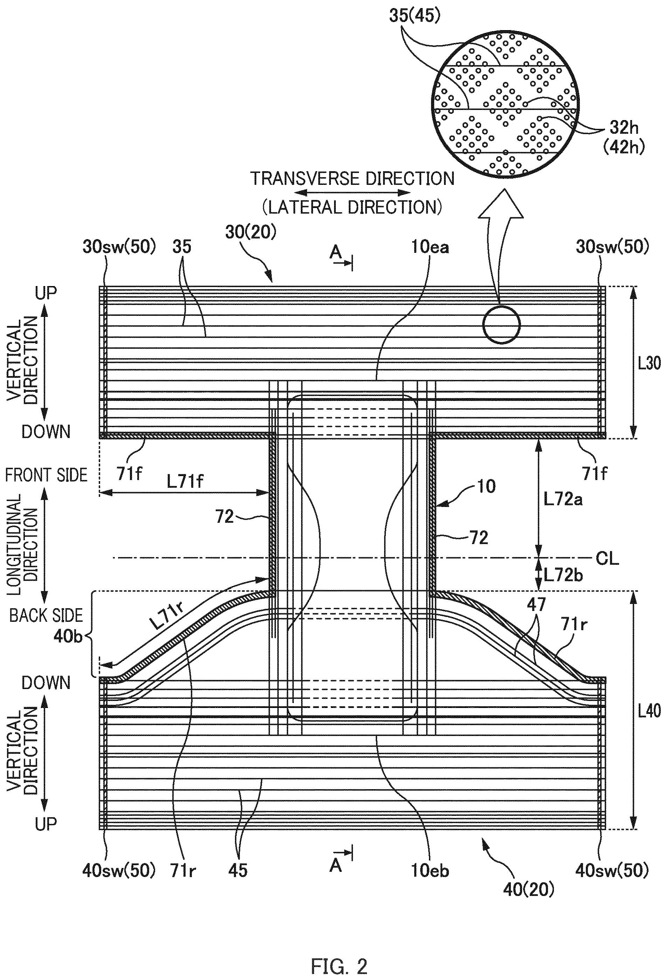

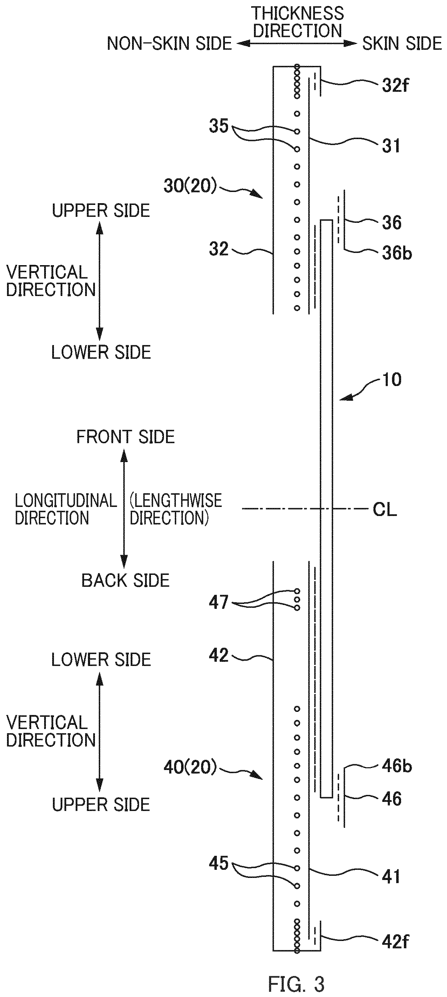

FIG. 1 is a schematic perspective view of a diaper 1. FIG. 2 is a plan view of the diaper 1 in an unfolded and stretched state. FIG. 3 is a schematic cross-sectional view taken along a line A-A in FIG. 2. It should be noted that the "stretched state" of the diaper 1 refers to a state where the entire diaper 1 (the entire product) is stretched without being wrinkled, specifically, a state where the diaper is stretched such that the dimensions of constituent members of the diaper 1 (e.g., an absorbent main body 10, a waist member 20, or the like to be described later) match or are close to the dimensions of the members on their own.

In an underpants-shaped state shown in FIG. 1, the diaper 1 has a vertical direction, a lateral direction, and a front-back direction that intersect with each other and has a waist opening BH and a pair of leg openings LH and LH. The upper side in the vertical direction corresponds to the waist opening BH side, and the lower side corresponds to the crotch side. In addition, the front side in the front-back direction corresponds to the wearer's front side, and the back side corresponds to the wearer's back side. Further, in an unfolded state in FIG. 2, the diaper 1 has a longitudinal direction and a transverse direction that intersect with each other. The longitudinal direction is a direction extending along the vertical direction in FIG. 1 and corresponds to the lengthwise direction of the absorbent main body 10. The transverse direction is a direction extending along the lateral direction in FIG. 1. In addition, as shown in FIG. 3, a direction in which constituent members of the diaper 1 are overlaid is referred to as a thickness direction. In the thickness direction, the side that comes into contact with the wearer's skin is defined as the skin side, and the side opposite to the skin side is defined as the non-skin side.

The diaper 1 has a liquid-absorbent absorbent main body 10, and a waist member 20 that is arranged on the non-skin side of the absorbent main body 10. The waist member 20 has a front waist portion 30 corresponding to the front panel of the diaper 1 and a back waist portion 40 corresponding to the back panel of the diaper 1. That is, the diaper 1 of the first embodiment is a so-called three-piece type underpants-shaped diaper including: as a first component, an absorbent main body 10 that is applied to the wearer's crotch portion and absorbs excrement such as urine or the like; as a second component, a front waist portion 30 that covers the wearer's stomach side portion; and as a third component, a back waist portion 40 that covers the wearer's back side portion.

In an unfolded state in FIG. 2, in a state where the front waist portion 30 and the back waist portion 40 are arranged side by side in the longitudinal direction with a space with respect to each other, lengthwise (longitudinal) end portions 10ea and 10eb of the absorbent main body 10 are respectively joined and fixed to the skin side of the nearest waist portions 30 and 40 while the absorbent main body 10 is spanned between the front waist portion 30 and the back waist portion 40. The external shape thereof forms a substantially H shape in a plan view. Then, from this state, the absorbent main body 10 is folded one time at a fold position, which is the longitudinal central position CL (doubling fold line). In this folded state, the front waist portion 30 and the back waist portion 40 that face each other are joined and connected to each other at two lateral side portions 30sw and 40sw to form a pair of side joining portions 50 and 50. That is, the front waist portion 30 and the back waist portion 40 are shaped into an annular shape by the pair of side joining portions 50 and 50. It should be noted that the side joining portion 50 is formed by commonly-known joining means such as welding or adhesive. Accordingly, the diaper 1 is in an underpants-shaped state in which the waist opening BH and the pair of leg openings LH and LH are formed as shown in FIG. 1. Further, at this time, the central position CL that is the doubling fold line of the diaper 1 serves as the lowermost end portion of the leg opening LH in the vertical direction. In the present specification, description will be made assuming that "the longitudinal central position CL in the unfolded state" of the diaper 1 and "the vertical lowermost end position of the diaper 1 in the underpants-shaped state" have the same meaning.

The leg openings LH of the diaper 1 are formed of parts of the waist member 20 and the absorbent main body 10 below the side joining portion 50 in the vertical direction (including the lower end of the side joining portion 50). Specifically, a region surrounded by the lower end edge portion of the front waist portion 30, the lower end edge portion of the back waist portion 40, and two lateral side edge portions of the absorbent main body 10 serves as the leg opening LH.

Hereinafter, in the region along the leg opening LH indicated by the hatched portion in FIG. 2, a region formed by the lower end edge portion of the front waist portion 30 will also be referred to as a front leg-circumferential region 71f, and a region formed by the lower end edge portion of the back waist portion 40 will also be referred to as a back leg-circumferential region 71r. The front leg-circumferential region 71f and the back leg-circumferential region 71r are combined with each other to form a "first region 71". Further, a region formed by the side edge portion of the absorbent main body 10 will also be referred to as a crotch leg-circumferential region 72. This crotch leg-circumferential region 72 is defined as a "second region 72". It should be noted that the "region along the leg opening LH" refers to a region having a predetermined width (e.g., a width of approximately 5 mm) toward the inside from the circumferential edge (edge) of the leg opening LH.

### Absorbent Main body 10

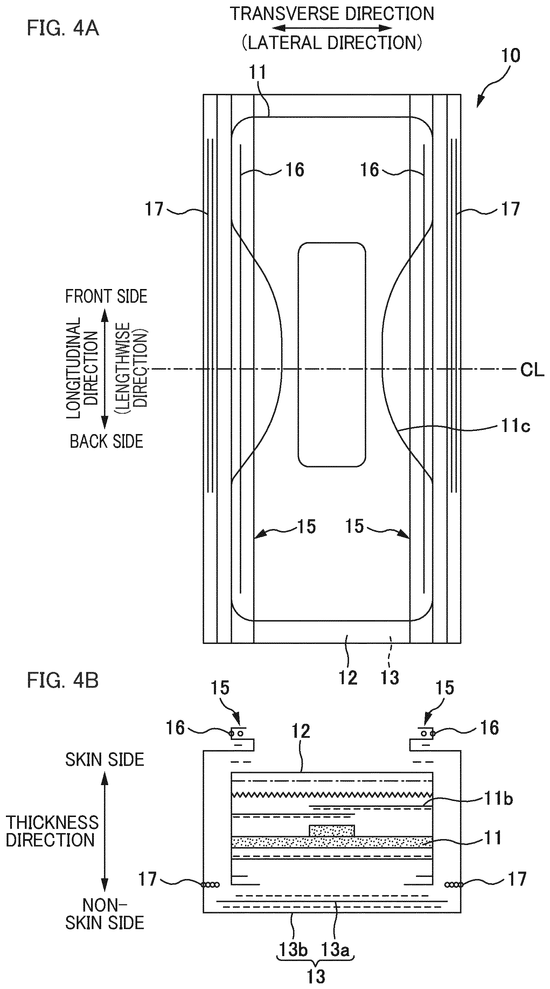

FIG. 4A is a plan view of the absorbent main body 10, and FIG. 4B is a schematic cross-sectional view of the absorbent main body 10.

The absorbent main body 10 has an absorbent core 11 that absorbs excreted fluids, a top sheet 12 that is arranged on the skin side in the thickness direction with respect to the absorbent core 11, and a back sheet 13 that is arranged on the non-skin side with respect to the absorbent core 11. However, the absorbent main body 10 may include other sheet members. For example, a second sheet (not shown) may be provided between the top sheet 12 and the absorbent core 11 in the thickness direction.

The absorbent core 11 is a member that absorbs and holds excreted fluid such as urine, and is formed of, for example, a liquid absorbent fiber (e.g., a pulp fiber) containing a superabsorbent polymer (SAP). It should be noted that the outer circumferential surface of the absorbent core 11 may be covered with a liquid-permeable sheet member (core-wrapping sheet 11b) such as tissue paper or nonwoven fabric. The absorbent core 11 of the present embodiment has a narrow portion 11c, which has a narrow width in the lateral direction, between a front end and a back end in the lengthwise direction, and has a substantially hourglass shape in a plan view as shown in FIG. 4A. This narrow portion 11c is a portion sandwiched between the wearer's two legs while the diaper 1 is put on, and the lateral length thereof is small (the width is narrow), making the absorbent core 11 easier to fit to the wearer's crotch. Further, in the present embodiment, as shown in FIG. 4B, the absorbent core 11 is in a state where two layers of the absorbent core are overlaid on each other in the thickness direction, but the shape of the absorbent core 11 is not limited thereto.

The top sheet 12 is a liquid-permeable sheet, and, for example, a hydrophilic air-through nonwoven fabric, spunbond nonwoven fabric, or the like may be used. In the present embodiment, as shown in FIG. 4B, two lateral side portions are folded back toward the non-skin side so as to enclose the absorbent core 11.

The back sheet 13 has a two-layer structure including a liquid-impermeable sheet 13a and a hydrophobic exterior sheet 13b arranged on the non-skin side of the liquid-impermeable sheet 13a. For example, as the liquid-impermeable sheet 13a, a resin film or the like is used, and as the exterior sheet 13b, a flexible hydrophobic nonwoven fabric sheet is used.

On two lateral side portions of the absorbent main body 10, a pair of the leak-proof wall portions 15 are respectively provided along the longitudinal direction (the lengthwise direction of the absorbent main body 10). In the present embodiment, the leak-proof wall portion 15 is formed by the above-described exterior sheet 13b. Specifically, in the lateral direction (transverse direction), a part of the exterior sheet 13b is folded toward the skin side at a plurality of locations as shown in FIG. 4B, while extending outward with respect to two end portions of the absorbent core 11, to form the pair of leak-proof wall portions 15. To the skin-side end portion (leading end portion) of each leak-proof wall portion 15, leak-proof-wall elastic members 16 such as elastic strings are attached in a state of being stretched along the longitudinal direction (the lengthwise direction of the absorbent main body 10). While the diaper 1 is put on, the leak-proof wall portions 15 rise toward the wearer's skin side and fit to the wearer's crotch portion due to the stretchability developed by the leak-proof-wall elastic members 16.

Further, to two lateral side portions of the absorbent main body 10, leg elastic members 17 such as elastic strings are attached in a state of being stretched along the longitudinal direction (the lengthwise direction of the absorbent main body 10). While the diaper 1 is put on, the two side portions of the absorbent main body 10 contract and becomes more likely to fit around the wearer's legs due to the stretchability developed by the leg elastic members 17.

### Front Waist Portion 30

As shown in FIG. 3, the front waist portion 30 includes: a skin-side sheet 31 that is arranged farthest on the skin side in the thickness direction; a non-skin-side sheet 32 that is overlaid so as to be adjacent to the non-skin side of the skin-side sheet 31; and waist elastic members 35 that are provided between the skin-side sheet 31 and the non-skin-side sheet 32 in the thickness direction. The front waist portion 30 basically has a two-layer structure constituted by the skin-side sheet 31 and the non-skin-side sheet 32, but may partially have a configuration of three or more layers including a skin surface sheet 36 or the like described below.

The skin-side sheet 31 and the non-skin-side sheet 32 are sheet members having a rectangular shape in a plan view as shown in FIG. 2, and are formed of, for example, an SMS nonwoven fabric or the like. It should be noted that in the diaper 1, the sheet member (nonwoven fabric sheet) that constitutes the non-skin-side sheet 32 has higher hydrophilicity than the sheet member (nonwoven fabric sheet) that constitutes the skin-side sheet 31. That is, the skin-side sheet 31 is formed of a nonwoven fabric sheet having low hydrophilicity (hereinafter, also referred to as a "hydrophobic nonwoven fabric"), and the non-skin-side sheet 32 is formed of a nonwoven fabric sheet having higher hydrophilicity than the skin-side sheet 31 (hereinafter, also referred to as a "hydrophilic nonwoven fabric"). Details of the hydrophilicity of each of the sheets 31 and 32 and the function based on the difference in hydrophilicity will be described later.

Further, on the surface of the non-skin-side sheet 32, the plurality of hole portions 32h as shown in a partially enlarged view of FIG. 2 are provided. The hole portions 32h are each a through hole that penetrates the non-skin-side sheet 32 in the thickness direction. Providing the hole portions 32h makes it possible to enhance the breathability of the front waist portion 30. Further, arranging the hole portions 32h visibly on the non-skin surface side of the front waist portion 30 makes the user more likely to invoke that the front waist portion 30 has good breathability. Each of the hole portions 32h may have, for example, a circular shape having a diameter of approximately 1 mm, but the shape and arrangement (number and pattern) of the hole portions 32h can be appropriately changed. It should be noted that, in the diaper 1, the through hole corresponding to the hole portion 32h is not provided in the skin-side sheet 31.

The front waist portion 30 of the present embodiment has a folded-back portion 32f in which the upper end portion (the front end portion in the longitudinal direction) of the non-skin-side sheet 32 is folded back from the non-skin side to the skin side and from the front side to the back side in the longitudinal direction. By covering a part (upper end portion) of the skin-side sheet 31 with the folded-back portion 32f, the upper end edge of the skin-side sheet 31 is prevented from biting into the wearer's skin. However, the folded-back portion 32f need not have to be necessarily provided.

Between the skin-side sheet 31 and the non-skin-side sheet 32, a plurality of waist elastic members 35 are arranged side by side in the vertical direction, and are attached in a state of being stretched in the lateral direction. The front waist portion 30 fits around the wearer's front waist due to the stretchability developed by the waist elastic member 35.

The waist elastic members 35 can be attached using an adhesive such as a hot-melt adhesive. For example, it is possible to attach the waist elastic members 35 by applying a hot-melt adhesive to each of them, stretching the waist elastic member 35 at a predetermined stretch factor, and sandwiching the waist elastic member 35 between the skin-side sheet 31 and the non-skin-side sheet 32. That is, the skin-side sheet 31 and the non-skin-side sheet 32 are joined using the adhesive with the waist elastic members 35 interposed therebetween. Further, the waist elastic members 35 may be attached by applying an adhesive to the skin-side sheet 31 side and the non-skin-side sheet 32 side, or the waist elastic member 35 may be attached by welding means in which a later-described welding portion 60 is used.

Further, the front waist portion 30 may have the skin surface sheet 36. As shown in FIG. 3, the skin surface sheet 36 is a sheet member arranged so as to cover the upper end portion (the front end portion in the longitudinal direction) of the absorbent main body 10 from the skin side, and functions as a cover sheet. Accordingly, the upper end edge of the absorbent main body 10 is prevented from biting into the wearer's skin while the diaper 1 is put on. The skin surface sheet 36 is formed of, for example, an SMS nonwoven fabric sheet or the like. It should be noted that the skin surface sheet 36 need not be necessarily provided.

In the front waist portion 30 of the present embodiment, in the case where a sheet member is provided on the skin side with respect to the skin-side sheet 31 or on the non-skin side with respect to the non-skin-side sheet 32, these sheets are arranged so as to cover only a part of the skin-side sheet 31 and the non-skin-side sheet 32. For example, the skin surface sheet 36 in FIG. 3 is provided so as to cover only a part of the skin-side sheet 31, and at least a part of the skin-side sheet 31 is in a state of being exposed to the wearer's skin side.

### Back Waist Portion 40

The back waist portion 40 has substantially the same configuration as the front waist portion 30. That is, the back waist portion 40 includes: a skin-side sheet 41 that is arranged farthest on the skin side in the thickness direction; a non-skin-side sheet 42 that is overlaid so as to be adjacent to the non-skin side of the skin-side sheet 41; and waist elastic members 45 that is provided between the skin-side sheet 41 and the non-skin-side sheet 42 in the thickness direction. Further, similar to the front waist portion 30, the back waist portion 40 may have hole portions 42h, a folded-back portion 42f, a skin surface sheet 46, and the like (see FIGS. 2 and 3). The configuration of the members is substantially the same as that of the front waist portion 30, and therefore description thereof is omitted.

On the other hand, the external shape of the back waist portion 40 is different from the external shape of the front waist portion 30. Specifically, as shown in FIG. 2, the back waist portion 40 has a buttocks cover 40b whose lower portion in the vertical direction with respect to the side joining portion 50 (side portion 40sw) has a substantially trapezoidal shape. The buttocks cover 40b is a portion whose lateral width is narrowed from the upper side to the lower side in the vertical direction and whose outer edge (corresponding to the back leg-circumferential region 71r) is curved. By providing the buttocks cover 40b, the back waist portion 40 can widely cover the wearer's buttocks while the diaper 1 is put on.

Further, in the buttocks cover 40b, curved elastic members 47 such as elastic strings as shown in FIG. 2 are provided. The curved elastic members 47 are each attached between the skin-side sheet 41 and the non-skin-side sheet 42 in a state of being stretched along the end edge portion of the buttocks cover 40b. The stretchability developed by the curved elastic members 47 makes the buttocks cover 40b of the back waist portion 40 likely to fit to the wearer's buttocks while the diaper 1 is put on, and also makes it difficult to be turned up from the buttocks.

### Hydrophilicity of Sheet Member

Here, the hydrophilicity of the sheet member will be described. In the diaper 1, a hydrophobic nonwoven fabric is used as the skin-side sheets 31 and 41 that constitute the waist member 20 (the front waist portion 30 and the back waist portion 40), and a hydrophilic nonwoven fabric having higher hydrophilicity than the hydrophobic nonwoven fabric is used as the non-skin-side sheets 32 and 42. Further, as the exterior sheet 13b that constitutes the absorbent main body 10, a hydrophobic nonwoven fabric is used.

The hydrophilic nonwoven fabric of the present embodiment has increased hydrophilicity by undergoing a treatment for attaching a predetermined oil agent (hydrophilic oil agent) to the hydrophobic nonwoven fabric (hydrophilic treatment). As the oil agent used in the hydrophilic treatment, it is possible to use commercially available oil agents having an effect as antistatic agents for fibers, such as anionic oil agents, nonionic oil agents, and blends thereof. These oil agents are put into an oil tank and then subjected to oiling with an oiling roller or the like. As a result, the hydrophilicity of the hydrophobic nonwoven fabric can be enhanced, obtaining the hydrophilic nonwoven fabric. However, the hydrophilic nonwoven fabric may be formed by other methods. For example, a hydrophilic nonwoven fabric may be obtained by manufacturing a nonwoven fabric using highly hydrophilic fibers.

It should be noted that, in the present embodiment, the entire nonwoven fabric that constitutes the non-skin-side sheet 32 is subject to the hydrophilic treatment, and the hydrophilicity of the entirety of the non-skin-side sheet 32 is enhanced. However, a configuration is possible in which the hydrophilicity is enhanced only in a partial region of the non-skin-side sheet 32. For example, the sheet member may have a locally high hydrophilic portion and a locally low hydrophilic portion by undergoing a hydrophilic treatment only on the partial region of the non-skin-side sheet 32.

The hydrophilicity of a sheet member can be evaluated by measuring a contact angle when ion exchange water is brought into contact with the surface of the sheet member. Specifically, in the case where the contact angle between the hydrophilic nonwoven fabric and the ion exchange water is smaller than the contact angle between the hydrophobic nonwoven fabric and the ion exchange water, the hydrophilicity of the hydrophilic nonwoven fabric becomes higher than the hydrophilicity of the hydrophobic nonwoven fabric. For the hydrophilic nonwoven fabric (non-skin-side sheet 32) used in the present embodiment, the contact angle with the ion exchange water is desirably less than 90 degrees, and more desirably 50 degrees or less. On the other hand, for the hydrophobic nonwoven fabric (skin-side sheet 31), the contact angle with ion exchange water is desirably 90 degrees or more, and more desirably 120 degrees or more.

Further, as described below, the magnitude of hydrophilicity can be compared by comparing the amounts of the hydrophilic oil agent contained per unit volume in the nonwoven fabric.

The contact angle can be measured by the following method using, for example, a contact angle meter MCA-J manufactured by Kyowa Interface Science Co., Ltd. First, ion exchange water is dropped (approximately 20 picoliters) onto the surface of the fibers that constitute a sheet member (sheet to be measured), and then immediately the contact angle is measured using the contact angle meter. The measurement is performed at a plurality of places (e.g., five or more places) on the surface of the sheet to be measured, and the average value of these positions is defined as the contact angle. It should be noted that the measurement environment temperature is set to 22°C.

Alternatively, the contact angle may be measured by capturing images of the sheet to be measured onto which ion exchange water is dropped from the cross-sectional direction of the sheet to be measured, analyzing the captured image, and measuring the angle between the ion exchange water droplet and the sheet to be measured.

### Moisture Absorption and Evaporation

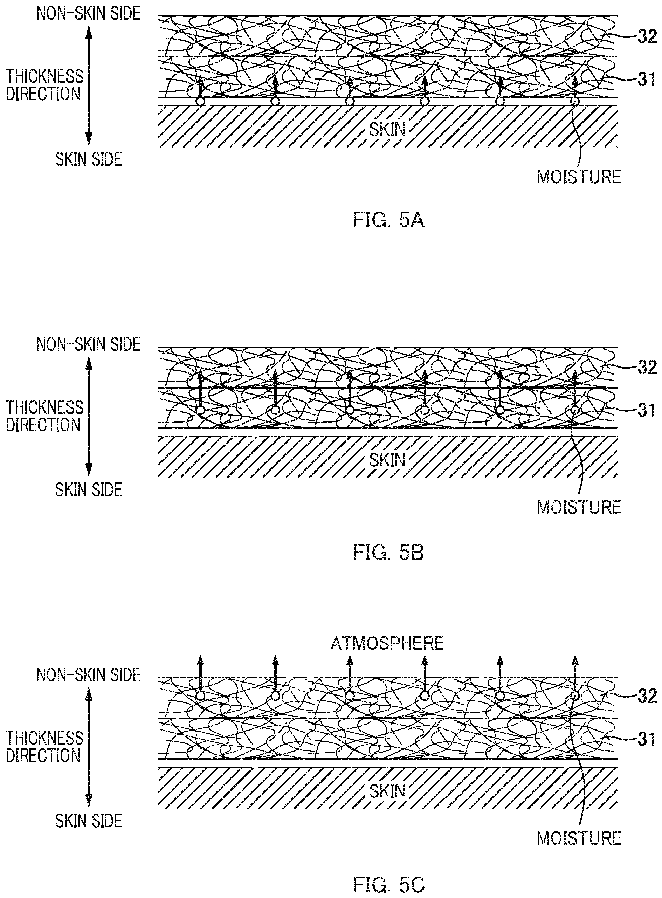

A mechanism of absorbing moisture such as sweat from the wearer's skin and evaporating the absorbed moisture to the atmosphere in the diaper 1 including the hydrophilic nonwoven fabric will be described. FIGS. 5A to 5C are explanatory diagrams illustrating the basic principle when moisture is absorbed and evaporated in the diaper 1. In FIG. 5, the cross-sections of the front waist portion 30 among the members that constitute the diaper 1 are schematically shown.

First, when the wearer puts on the diaper 1, the skin-side sheet 31 (hydrophobic nonwoven fabric) arranged on the skin side of the front waist portion 30 in the thickness direction comes into contact with the wearer's skin, as shown in FIG. 5A. As described above, the skin-side sheet 31 of the present embodiment is formed of SMS nonwoven fabric and includes meltblown fibers having a small fiber diameter compared with spunbond fibers. Therefore, the thin meltblown fibers have a portion in which the inter-fiber distance becomes narrow by being densely entangled. According to the above-described configuration, a capillary phenomenon occurs in the skin-side sheet 31, and moisture such as sweat attached to the wearer's skin is easily absorbed.

Next, as shown in FIG. 5B, the moisture absorbed by the skin-side sheet 31 is transferred to the non-skin-side sheet 32 (hydrophilic nonwoven fabric) that is overlaid to be adjacent to the non-skin side of the skin-side sheet 31. This is because, while the skin-side sheet 31 is formed of a hydrophobic nonwoven fabric sheet, the non-skin-side sheet 32 is formed of a hydrophilic nonwoven fabric sheet, a difference in the magnitude of hydrophilicity (hydrophilic gradient) is thus generated between the two sheets, and this makes moisture more likely to move from the low hydrophilic skin-side sheet 31 side to the highly hydrophilic non-skin-side sheet 32 side. Therefore, moisture is less likely to be held on the skin-side sheet 31 side that is in contact with the wearer's skin, and is more likely to be held on the non-skin-side sheet 32 side that is not in contact with the wearer's skin.

The moisture held in the non-skin-side sheet 32 is evaporated into the atmosphere from the non-skin-side surface of the non-skin-side sheet 32. As shown in FIG. 5C, in the case where no other member (sheet member) is arranged on the non-skin side surface of the non-skin-side sheet 32, the entirety of the non-skin-side surface of the non-skin-side sheet 32 faces the atmosphere. That is, the area of the interface between the non-skin-side sheet 32 and the atmosphere is increased, and evaporation of moisture is likely to occur.

In this manner, in the waist member 20 of the diaper 1, the moisture absorbed from the wearer's skin by the hydrophobic nonwoven fabric is transferred to the hydrophilic nonwoven fabric on the non-skin side, and the moisture is released into the atmosphere from the non-skin-side surface of the hydrophilic nonwoven fabric. That is, moisture can be absorbed and efficiently evaporated to the atmosphere.

Accordingly, moisture is less likely to come into contact with the wearer's skin, and this makes it possible to suppress the occurrence of skin problems such as rashes caused by the wet skin of the wearer or for the wearer to be less likely to feel discomfort.

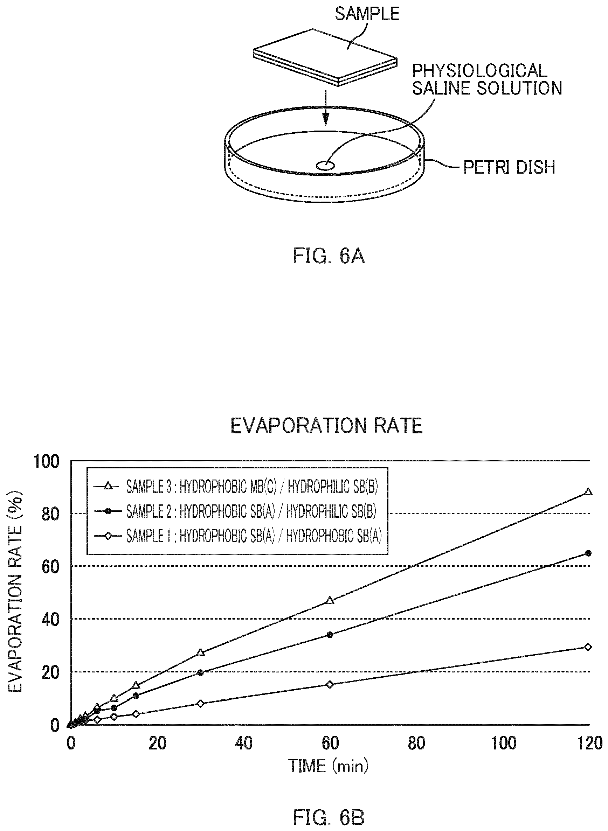

The results of verification experiments on such evaporation of moisture are shown in FIG. 6. FIG. 6A is an explanatory diagram illustrating an experimental method for measuring the evaporation rate. FIG. 6B is a graph showing evaporation rate data obtained from experimental results.

The experiment was carried out according to the evaporation property (II) test defined in ISO 17617, Method B. First, two types of sheet members are overlaid on each other and are cut to a predetermined size, preparing three types of sample (test pieces) 1 to 3. Sample 1 is a piece in which two layers of hydrophobic spunbond nonwoven fabric A (basis weight: 15 gsm) are overlaid on each other. Sample 2 is a piece in which a hydrophilic spunbond nonwoven fabric B (basis weight: 20 gsm) is overlaid on and above the spunbond nonwoven fabric A. Sample 3 is a piece in which the hydrophilic spunbond nonwoven fabric B (basis weight: 20 gsm) is overlaid on and above a hydrophobic meltblown nonwoven fabric C (basis weight: 13 gsm).

Next, as shown in FIG. 6A, 1 cc of physiological saline solution is dropped onto the petri dish to form water droplets, and the sample is arranged so as to cover the water droplet from the top. Immediately after the sample is arranged, the total of the weights of the sample and the petri dish is measured and recorded. Thereafter, the total of the weights of the sample and the petri dish is measured every time a predetermined time has elapsed, and the rate of decrease from the initially measured weight is calculated as the evaporation rate of moisture (physiological saline solution). This experiment was performed on each of three types of sample.

As a result, as shown in FIG. 6B, it was clarified that Sample 1 in which two layers of hydrophobic nonwoven fabric were overlaid on each other had the lowest evaporation rate. Further, when comparing Sample 1 and Sample 2, it was confirmed that Sample 2 in which the hydrophilic nonwoven fabric was overlaid on the hydrophobic nonwoven fabric had a higher evaporation rate than Sample 1. This indicates that, as described above, by arranging the hydrophilic nonwoven fabric on the side that comes into contact with the atmosphere (non-skin side), moisture is transferred from the hydrophobic nonwoven fabric to the hydrophilic nonwoven fabric, promoting the evaporation of moisture from the hydrophilic nonwoven fabric to the atmosphere.

Further, when comparing Sample 2 and Sample 3, it was confirmed that Sample 3 using a meltblown nonwoven fabric having a small fiber diameter as the hydrophobic nonwoven fabric had a higher evaporation rate than Sample 2 using a spunbond nonwoven fabric having a large fiber diameter. This indicates that, as described above, the higher the density of fibers that constitute the hydrophobic nonwoven fabric, the more likely the capillary phenomenon is to occur, making the hydrophobic nonwoven fabric more likely to absorb moisture.

In the diaper 1 of the first embodiment, in at least a region in the waist member 20 that overlap the pair of side joining portions 50 with respect to the vertical direction, the hydrophilic nonwoven fabric is overlaid on and adjacent to the non-skin side of the hydrophobic nonwoven fabric. That is, the hydrophobic region is provided in a surface located farthest on the skin side, and the hydrophilic region is provided in a surface located farthest on the non-skin side. Therefore, as shown in the experimental results of FIG. 6, the diaper 1 has a structure in which moisture is likely to be absorb from the skin side and evaporated toward the non-skin side.

It should be noted that in the diaper 1, the waist elastic members 35 are provided between the skin-side sheet 31 and the non-skin-side sheet 32 (see FIG. 3). When the wearer moves the body in a state where the diaper 1 is put on, the waist elastic members 35 stretch and contract in the lateral direction, and in conjunction with the stretching and contracting of the waist elastic members 35, the skin-side sheet 31 and the non-skin-side sheet 32 also stretch and contract in the lateral direction. Accordingly, the effect of absorbing and evaporating moisture can be further enhanced.

For example, when the waist elastic members 35 contract, the skin-side sheet 31 contracts, increasing the fiber density and decreasing the inter-fiber distance. In this case, the capillary phenomenon is more likely to occur, and moisture is likely to be absorbed. On the other hand, when the waist elastic members 35 are stretched, the skin-side sheet 31 and the non-skin-side sheet 32 are stretched and wrinkles and the like on the sheet surface are stretched. Therefore, the skin-side sheet 31 and the non-skin-side sheet 32 comes into close contact with each other, and moisture transfer based on a hydrophilic gradient is likely to occur. Further, when the non-skin-side sheet 32 is stretched, moisture is likely to be diffused in the planar direction of the non-skin-side sheet 32, promoting evaporation to the atmosphere.

### Leg Openings

It has been described that the waist member 20 of the diaper 1 efficiently absorbs and evaporates moisture, making the wearer's waist less likely to fees discomfort. However, when the leg openings LH are wet while the diaper 1 is put on, there is a risk of causing discomfort in the wearer's crotch portion.

The leg opening LH of the diaper 1 includes a front leg-circumferential region 71f and a back leg-circumferential region 71r (first region 71) formed by the waist member 20 (30 and 40), and a crotch leg-circumferential region 72 (second region 72) formed by the absorbent main body 10 (see FIG. 2). In the first region 71 of the above-described regions, while the diaper 1 is put on, the hydrophobic skin-side sheets 31 and 41 come into contact with the wearer's skin, and the hydrophilic non-skin-side sheets 32 and 42 do not come into contact with the wearer's skin (see FIG. 5). Therefore, the wearer's skin is less likely to become wet and discomfort is less likely to be caused.

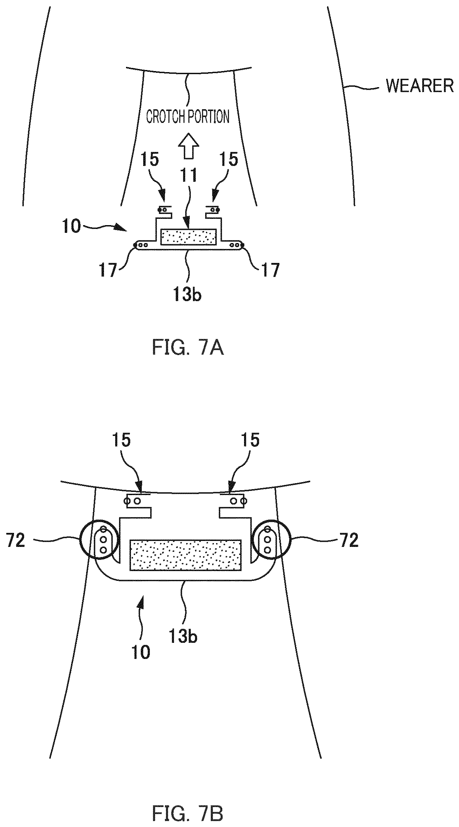

In contrast, in the second regions 72, the non-skin-side surface of the exterior sheet 13b that constitutes the absorbent main body 10 comes into contact with the wearer's body. FIGS. 7A and 7B are diagrams illustrating how the absorbent main body 10 fits to the crotch portion of a wearer while the diaper 1 is put on. When putting on the diaper 1, the absorbent main body 10 (diaper 1) is pulled up along the wearer's legs from the lower side (toe side) to the upper side (crotch side) in the vertical direction as shown in FIG. 7A. In FIG. 7A, the portions that protrude on two lateral sides of the absorbent main body 10 (portions in which the leg elastic members 17 are arranged) are portions corresponding to the crotch leg-circumferential regions 72 (second region 72).

In the absorbent main body 10 pulled up to the crotch portion, as shown in FIG. 7B, the leading ends of the leak-proof wall portions 15 rise toward the skin side due to the stretchability developed by the leak-proof-wall elastic members 16 and come into contact with the wearer's crotch (groin portion). Further, the second regions 72 come into contact with the wearer's legs respectively so as to be pressed against the legs due to the stretchability developed by the leg elastic members 17. That is, in the crotch portion of the leg opening LH of the diaper 1, the exterior sheet 13b, which is a sheet member arranged farthest on the non-skin side of the absorbent main body 10, comes into contact with the wearer's skin.

Therefore, in the case where the exterior sheet 13b of the absorbent main body 10 is formed of a hydrophilic nonwoven fabric similar to the non-skin-side sheets 32 and 42 of the waist member 20, the second region 72 holds moisture and likely to be wet. In this case, there is a risk that the wearer is likely to be wet around the legs in the crotch portion, making the wearer feel discomfort.

In contrast, in the diaper 1 of the first embodiment, the exterior sheet 13b is formed of a hydrophobic nonwoven fabric. That is, in the leg openings LH, the hydrophobic nonwoven fabric is arranged farthest on the non-skin side of the second regions 72. This makes at least the non-skin side of a portion located at the lowermost end of the leg opening LH that comes into contact with the wearer's crotch portion (at the central position CL in the longitudinal direction) be a hydrophobic region, and the region has a structure in which moisture is less likely to be held. Accordingly, while the diaper 1 is put on, the wearer's skin is less likely to become wet in the wearer's crotch portion.

That is, in an underpants-shaped diaper having a so-called three-piece structure (see FIG. 2) like the diaper 1, by using a hydrophobic nonwoven fabric as the sheet member of the outermost layer of the absorbent main body 10 (exterior sheet 13b) that comes into contact with the wearer's crotch portion during usage, the wearer can be less likely to feel discomfort around the legs.

More specifically, in the leg openings LH of the three-piece type diaper 1, in the first region 71 formed of the waist member 20, the non-skin side is a hydrophilic region, and, in the second region 72 formed of the absorbent main body 10, the non-skin side is a hydrophobic region. In such a case, in the leg opening LH, moisture is likely to be evaporated in the first region 71 moisture is less likely to hold in the second region 72 that comes into contact with the wearer's crotch portion, making the wearer's crotch portion less likely to become wet. Accordingly, the wearer can be less likely to feel discomfort in a wide range of the crotch portion.

At this time, as shown in FIG. 2, it is preferable that the length L72a of the second region 72 (the length of the portion along the second region 72) on the front side with respect to the central position CL in the longitudinal direction (the lengthwise direction of the absorbent main body 10) is longer than the length L72b of the second region 72 (the length of the portion along the second region 72) on the back side with respect to the central position CL (L72a > L72b). While the diaper 1 is put on, there are many opportunities to move the wearer's legs forward, such as when the wearer walks. Therefore, by making elongated the length L72a of the second region 72 on the front side with respect to the central position CL, the hydrophobic region on the front side of the leg opening LH becomes wider, making the legs less likely to become wet even in the case where the wearer moves the legs forward. Accordingly, the wearer can be less likely to feel discomfort while the diaper is put on.

Further, it is preferable that in the first region 71 that forms the leg opening LH, the length L71r of the back leg-circumferential region 71r (the length of the portion along the region 71r) on the back side with respect to the central position CL in the longitudinal direction is longer than the length L71f of the front leg-circumferential region 71f (the length of the portion along the region 71f) on the front side with respect to the central position CL (L71r > L71f). The back leg-circumferential region 71r is a region that is likely to come into wide contact with the wearer's buttocks and that is likely to be pressed against the wearer's body due to the body pressure which is to be applied in a case such that the wearer sits down in a state where the wearer puts on the diaper **1.** Therefore, by making elongated the length L71r of the back leg-circumferential region 71r, the hydrophobic region on the back side of the leg opening LH (buttocks side), and the buttocks are less likely to become wet even in the case where the wearer takes a seated posture. Accordingly, the wearer can be less likely to feel discomfort while the diaper is put on.

Further, it is preferable that the waist member 20 (30, 40) of the diaper 1 has a two-layer structure in which two sheet members are overlaid in the thickness direction, namely a single skin-side sheet 31 (41) formed of hydrophobic nonwoven fabric and a single non-skin-side sheet 32 (42) formed of hydrophilic nonwoven fabric. Not providing extra sheet member in the waist member 20 makes it less likely to inhibit the absorption and evaporation of moisture mentioned above, and this makes it possible to more efficiently evaporate the moisture. The waist member 20 of the present embodiment partially has a three-layer structure including a folded-back portion 32f or the like shown in FIG. **3****.** However, the two-layer structure in which two sheet members, that is, the skin-side sheets 31 and 41 and the non-skin-side sheets 32 and 42, are overlaid on each other in the most region of the waist member 20, and the moisture can be efficiently evaporated. Further, the hydrophilic region provided on the non-skin-side surface of the waist member 20 is formed of one sheet of hydrophilic nonwoven fabric, and therefore the hydrophilic region is formed in a wide range of the waist member 20, which makes it possible to further enhance evaporation property.

However, this does not exclude providing another sheet member on the non-skin side with respect to the hydrophilic nonwoven fabric (non-skin-side sheets 32 and 42) in the waist member 20. For example, the skin-side sheet 31 (hydrophobic nonwoven fabric) may be folded back toward the non-skin side and overlaid so as to cover a part of the non-skin-side surface of the non-skin-side sheet 32. Even in such a case, the moisture evaporation effect is maintained in the portion in which the non-skin-side surface is not covered, and this can make the wearer less likely to feel discomfort.

Further, as shown in FIG. 2, it is preferable that the vertical length L40 of the back waist portion 40 is longer than the vertical length L30 of the front waist portion 30. Since the back waist portion 40 is a portion that covers the wearer's buttocks while the diaper 1 is put on, it is desirable that the buttocks are securely covered by increasing the area as much as possible. On the other hand, it is desirable that the area of the front waist portion 30 is not excessively increased so as not to interfere with the movement of the legs when the wearer moves the legs forward when walking or the like. Further, generally, the wearer is more likely to sweat on the back side compared with the front side, and the amount of sweating on the back-side is likely to large. Therefore, it is desirable that the area of the back waist portion 40 is large, making sweat on the back-side likely to be evaporated.

Further, since the back waist portion 40 of the diaper 1 has the buttocks cover 40b below the side joining portions 50 in the vertical direction, moisture such as sweat is likely to be evaporated by the buttocks cover 40b in the wearer's buttocks. Further, the buttocks cover 40b has the curved elastic members 47 extending along the end edge portions of the buttocks cover 40b (back leg-circumferential region 71r). The stretchability of the curved elastic members 47 makes the buttocks cover 40b likely to fit to the wearer's buttocks while the diaper 1 is put on. Further, since stretchability acts along the back leg-circumferential region 71r, the back leg-circumferential region 71r is less likely to turn up, enabling to make the wearer less likely to feel unpleasantness or discomfort and to make it likely to exhibit the moisture evaporation function by the back waist portion 40.

### Modified Example of Method for Attaching Waist Elastic Members 35 and 45

In the above-described embodiment, the waist elastic members 35 and 45 are attached to the waist member 20 by adhering means using a hot-melt adhesive or the like, but the method for attaching the waist elastic members 35 and 45 is not limited thereto. For example, the waist elastic members 35 and 45 may be attached to the waist member 20 using welding means such as ultrasonic welding. It should be noted that, since ultrasonic welding is a commonly-known technique, the description of ultrasonic welding is omitted in the present specification.

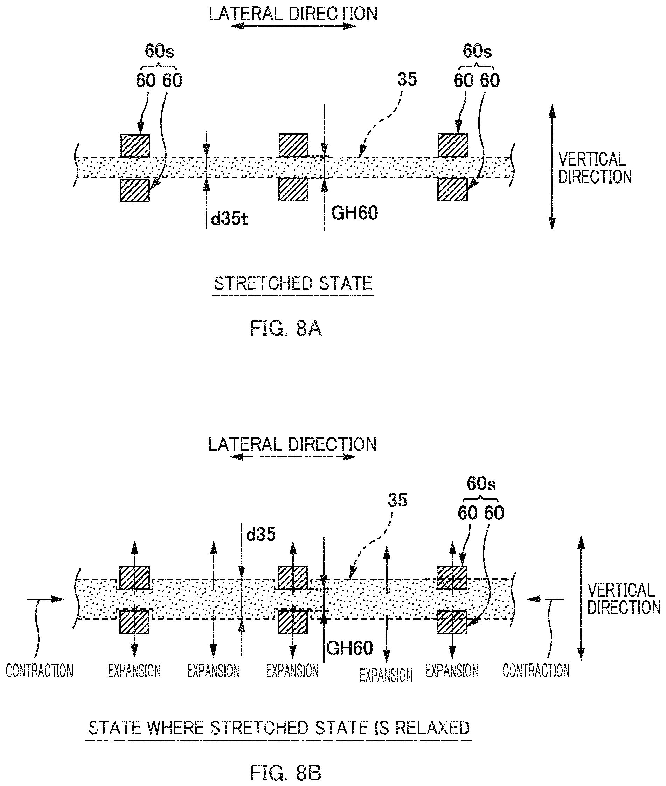

FIGS. 8A and 8B are explanatory diagrams illustrating a method for attaching the waist elastic member 35 to the front waist portion 30 using the welding portions 60. In the present modified example, the waist elastic member 35 is attached to the front waist portion 30 by a plurality of welding portions 60, 60, ..., which are discretely arranged in the lateral direction and the vertical direction. Each of the welding portions 60 is formed into a substantially rectangular shape by ultrasonic welding, and joins the skin-side sheet 31 and the non-skin-side sheet 32 of the front waist portion 30 in the thickness direction. The waist elastic member 35 is attached to the front waist portion 30 by sandwiching the waist elastic member 35 from two vertical sides between welding portion pairs 60s each of which is formed of two welding portions 60 and 60 that are adjacent to each other in the vertical direction.

As shown in FIG. 8A, a pair of welding portions 60 and 60 that constitute the welding portion pair 60s are arranged side by side in the vertical direction with a space GH60. The size of the space GH60 is set to be the same size as or slightly larger than the diameter d35t of the waist elastic member 35 in a state where the waist elastic member 35 is stretched to a predetermined stretch factor (GH60 ≥ d35t). That is, the waist elastic member 35 in the stretched state is arranged between the welding portion pair 60s in the vertical direction.

Next, when the waist elastic member 35 is relaxed from the stretched state, as shown in FIG. 8B, the waist elastic member 35 expands in the vertical direction while contracting in the lateral direction, and the diameter d35 in the natural state becomes larger than the vertical space GH60 of the welding portion pair 60s (d35 > GH60). Accordingly, the waist elastic member 35 is sandwiched between the welding portions 60 and 60 in the vertical direction. As a result, the waist elastic member 35 is attached to the front waist portion 30.

It should be noted that in the diaper 1 in the underpants-shaped state in FIG. 1, the waist elastic members 35 (45) are in a natural state where the waist elastic members 35 (45) are relaxed from the above-described stretched state. Further, in the diaper 1 in the underpants-shaped state, the waist elastic members 35 (45) are joined to the front waist portion 30 (back waist portion 40) by the side joining portions 50 and 50 on two lateral side portions. Therefore, even when the front waist portion 30 (back waist portion 40) is stretched in the lateral direction while the diaper 1 is put on, the waist elastic members 35 (45) are not detached from the waist member 20.

### Second Embodiment

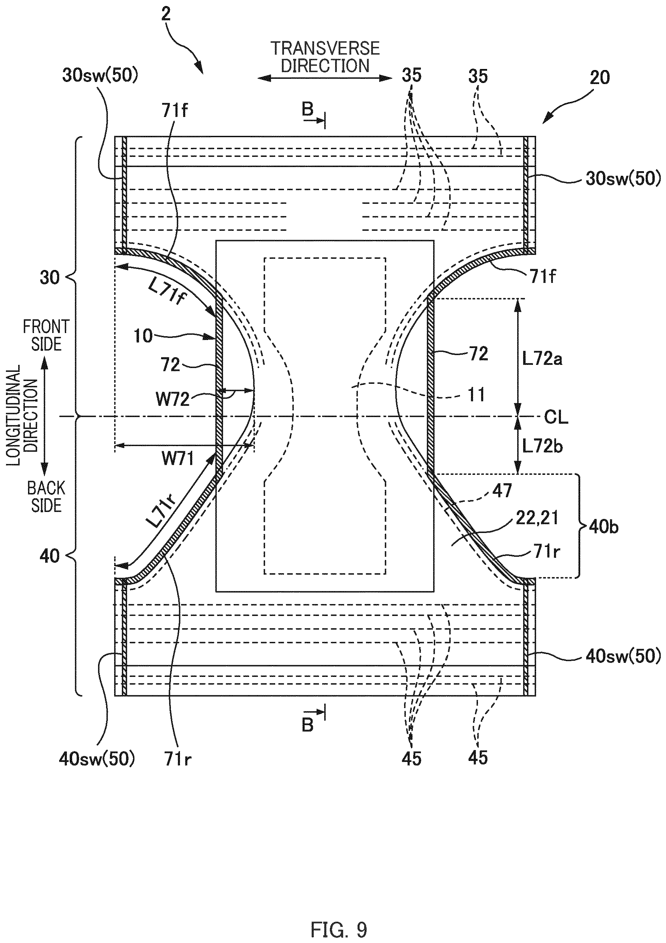

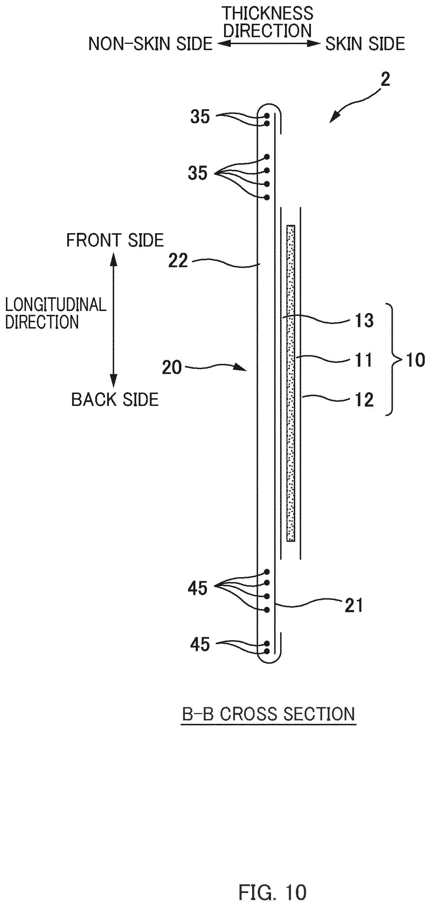

In a second embodiment, an underpants-shaped diaper 2 (hereinafter, also referred to as a "diaper 2") having a configuration partially different from that of the first embodiment will be described. FIG. 9 is a plan view of the diaper 2 in the unfolded and stretched state. FIG. 10 is a schematic cross-sectional view taken along a line B-B in FIG. 9. It should be noted that the directions (e.g., the longitudinal direction, the transverse direction, or the like) in FIGS. 9 and 10 is the same as the directions in the first embodiment.

The diaper 2 of the second embodiment has the liquid-absorbent absorbent main body 10, and the waist member 20 that is joined to the non-skin side of the absorbent main body 10. However, in the diaper 2, the front waist portion 30 and the back waist portion 40 are integrally constituted so as to be continuous in the longitudinal direction in FIGS. 9 and 10 (in the front-back direction). That is, the diaper 2 is a so-called two-piece type disposable diaper formed of the absorbent main body 10 serving as an interior body and the waist member 20 serving as an exterior body.

When shaping the diaper 2 in the unfolded state in FIG. 9 into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, similar to the diaper 1 in FIG. 1, the diaper 2 is in an underpants-shaped state having a waist opening BH and a pair of leg openings LH and LH.

In the diaper 2, the basic configuration and function of the absorbent main body 10 are substantially the same as those of the absorbent main body 10 of the diaper 1 of the first embodiment, and therefore description thereof is omitted. However, in the diaper 2, the crotch portion including the central position CL in the longitudinal direction (the lengthwise direction of the absorbent main body 10) has a portion in which the lateral width of the absorbent main body 10 is wider than the lateral width of the waist member 20. In other words, in the lateral direction, the minimum value of the length (width) of the waist member 20 is smaller than the minimum value of the length (width) of the absorbent main body 10. Therefore, during usage of the diaper 1, in the region in the vicinity of the wearer's crotch portion, two lateral side edge portions of the absorbent main body 10 come into contact with the wearer's legs.

That is, in the diaper 2, in the regions along the leg openings LH indicated by the hatched portion in FIG. 9, regions formed by the side edge portions of the absorbent main body 10 are the crotch leg-circumferential regions 72 (second region 72). On the other hand, the regions formed by the lower end edge portions of the front waist portion 30 are the front leg-circumferential regions 71f (first region 71), and the regions formed by the lower end edge portions of the back waist portion 40 are the back leg-circumferential regions 71r (first region 71). It should be noted that, a configuration is possible in which elastic members that stretch and contract in the longitudinal direction are provided on the side edge portion of the absorbent main body 10, and the side edge portions constitute so-called leg gathers that are wide in the lateral direction. In this case, two end portions of the leg gathers are side edge portions of the absorbent main body 10.

Further, in the diaper 2, a portion of the waist member 20 located on the front side with respect to the central position CL in the longitudinal direction (the lengthwise direction of the absorbent main body 10) serves as the front waist portion 30, and a portion located on the back side with respect to the central position CL serves as the back waist portion 40. The waist member 20 includes: a skin-side sheet 21; a non-skin-side sheet 22 that is overlaid to be adjacent to the non-skin side of the skin-side sheet 21; and waist elastic members 35 and 45 that are arranged between the skin-side sheet 21 and the non-skin-side sheet 22 in the thickness direction (see FIG. 10).

The skin-side sheet 21 is a hydrophobic nonwoven fabric sheet similar to the skin-side sheets 31 and 41 of the diaper 1. The non-skin-side sheet 22 is a hydrophilic nonwoven fabric sheet similar to the non-skin-side sheets 32 and 42 of the diaper 1. That is, in the diaper 2, the non-skin-side sheet 22 is nonwoven fabric having higher hydrophilicity than the skin-side sheet 21. Then, similar to the diaper 1, the waist elastic members 35 and 45 are formed of elastic strings or the like, and are attached between the skin-side sheet 21 and the non-skin-side sheet 22 in a state of being stretched in the lateral direction.

In the diaper 2 of the second embodiment, similar to the diaper 1, in at least a region in the waist member 20 that overlap the pair of side joining portions 50 with respect to the vertical direction, a hydrophobic region formed of a hydrophobic nonwoven fabric (skin-side sheet 21) is provided in a surface located farthest on the skin side, and a hydrophilic region formed of a hydrophilic nonwoven fabric (non-skin-side sheet 22) is provided in a surface located farthest on the non-skin side. Therefore, similar to the diaper 1 described above, the diaper 2 has a structure in which moisture is likely to be absorb from the wearer's skin side and evaporated into the atmosphere on the non-skin side.

In the diaper 2, the exterior sheet 13b of the absorbent main body 10 is formed of a hydrophobic nonwoven fabric. That is, in the leg openings LH, the hydrophobic nonwoven fabric is arranged farthest on the non-skin side of the second regions 72. This makes at least the non-skin side of a portion located at the lowermost end of the leg opening LH that comes into contact with the wearer's crotch portion (at the central position CL in the longitudinal direction) be a hydrophobic region, and the region has a structure in which moisture is less likely to be held. Accordingly, while the diaper 2 is put on, the wearer's skin is less likely to become wet in the crotch portion around the wearer's legs.

That is, in an underpants-shaped diaper having a so-called two-piece structure like the diaper 2, by using a hydrophobic nonwoven fabric as the sheet member of the outermost layer of the absorbent main body 10 (exterior sheet 13b) that comes into contact with the wearer's crotch portion, the wearer can be less likely to feel discomfort around the legs.

In the leg opening LH of the two-piece type diaper 2, in the first region 71 formed of the waist member 20, the non-skin side is a hydrophilic region, and, in the second region 72 formed of the absorbent main body 10, the non-skin side is a hydrophobic region. In such a case, in the leg opening LH, moisture is likely to be evaporated in the first region 71 moisture is less likely to hold in the second region 72 that comes into contact with the wearer's crotch portion, making the wearer's crotch portion less likely to become wet. Accordingly, the wearer can be less likely to feel discomfort in a wide range of the crotch portion.

Further, as shown in FIG. 9, it is preferable that the length L72a of the second region 72 (the length of the portion along the second region 72) on the front side with respect to the central position CL in the longitudinal direction (the lengthwise direction of the absorbent main body 10) is longer than the length L72b of the second region 72 (the length of the portion along the second region 72) on the back side with respect to the central position CL (L72a > L72b). While the diaper 2 is put on, there are many opportunities to move the wearer's legs forward, such as when the wearer walks. Therefore, by making elongated the length L72a of the second region 72 on the front side with respect to the central position CL, the hydrophobic region on the front side of the leg opening LH becomes wider, making the legs less likely to become wet even in the case where the wearer moves the legs forward. Accordingly, the wearer can be less likely to feel discomfort while the diaper is put on.

Further, it is preferable that in the first region 71 that forms the leg opening LH, the length L71r of the back leg-circumferential region 71r (the length of the portion along the region 71r) on the back side with respect to the central position CL in the longitudinal direction is longer than the length L71f of the front leg-circumferential region 71f (the length of the portion along the region 71f) on the front side with respect to the central position CL (L71r > L71f). The back leg-circumferential region 71r is a region that is likely to come into wide contact with the wearer's buttocks and that is likely to be pressed against the wearer's body due to the body pressure which is to be applied in a case such that the wearer sits down in a state where the wearer puts on the diaper 1. Therefore, by making elongated the length L71r of the back leg-circumferential region 71r, the hydrophobic region on the back side of the leg opening LH (buttocks side), and the buttocks are less likely to become wet even in the case where the wearer takes a seated posture. Accordingly, the wearer can be less likely to feel discomfort while the diaper is put on.

Further, in the lateral direction of the diaper 2, the distance between the outermost end of the waist member 20 and the innermost end of the waist member 20 is represented by W71, and the distance between the outermost end of the absorbent main body 10 and the innermost end of the waist member 20 is represented by W72 (see FIG. 9). This distance W72 represents the length (width) of a portion of the absorbent main body 10 that extends outward in the lateral direction from a portion where the lateral width of the waist member 20 is narrowest. Further, at this time, it is desirable that the width W72 is smaller than 1/2 of the width W71 (W71 × 1/2 > W72).

In the case where the side edge portions of the absorbent main body 10 extends outward beyond by more than 1/2 of the width W71, the opening area of the leg opening LH is reduced, and there is a risk that while the diaper 2 is put on, it makes the wearer difficult to move the legs or it makes the wearer feel discomfort because the crotch leg-circumferential region 72 is pressed against the wearer's groin. In contrast, in the case where the above-described relationship (W71 x 1/2 > W72) is satisfied, the side edge portions of the absorbent main body 10 (crotch leg-circumferential region 72) are prevented from excessively extend outward in the lateral direction. Accordingly, while the diaper 2 is put on, the wearer can easily move the legs and be less likely to feel discomfort around the legs.

Further, it is preferable that the waist member 20 (30, 40) of the diaper 2 has a two-layer structure in which two sheet members are overlaid in the thickness direction, namely a single skin-side sheet 21 formed of hydrophobic nonwoven fabric and a single non-skin-side sheet 22 formed of hydrophilic nonwoven fabric. Not providing extra sheet member in the waist member 20 makes it less likely to inhibit the absorption and evaporation of moisture, and this makes it possible to more efficiently evaporate the moisture. Further, the hydrophilic region provided on the non-skin-side surface of the waist member 20 is formed of one sheet of hydrophilic nonwoven fabric, and therefore the hydrophilic region is formed in a wide range of the waist member 20, which makes it possible to further enhance evaporation property.

Further, the back waist portion 40 of the diaper 2 has the buttocks cover 40b below the side joining portions 50 in the vertical direction (see FIG. 9). Moisture such as sweat can be likely to be evaporated by the buttocks cover 40b in the wearer's buttocks. Further, the buttocks cover 40b has the curved elastic members 47 extending along the end edge portions of the buttocks cover 40b (back leg-circumferential region 71r). The stretchability of the curved elastic members 47 makes the buttocks cover 40b likely to fit to the wearer's buttocks while the diaper 2 is put on. Further, since stretchability acts along the back leg-circumferential region 71r, the back leg-circumferential region 71r is less likely to turn up, enabling to make the wearer less likely to feel unpleasantness or discomfort and to make it likely to exhibit the moisture evaporation function by the back waist portion 40.

### Third Embodiment

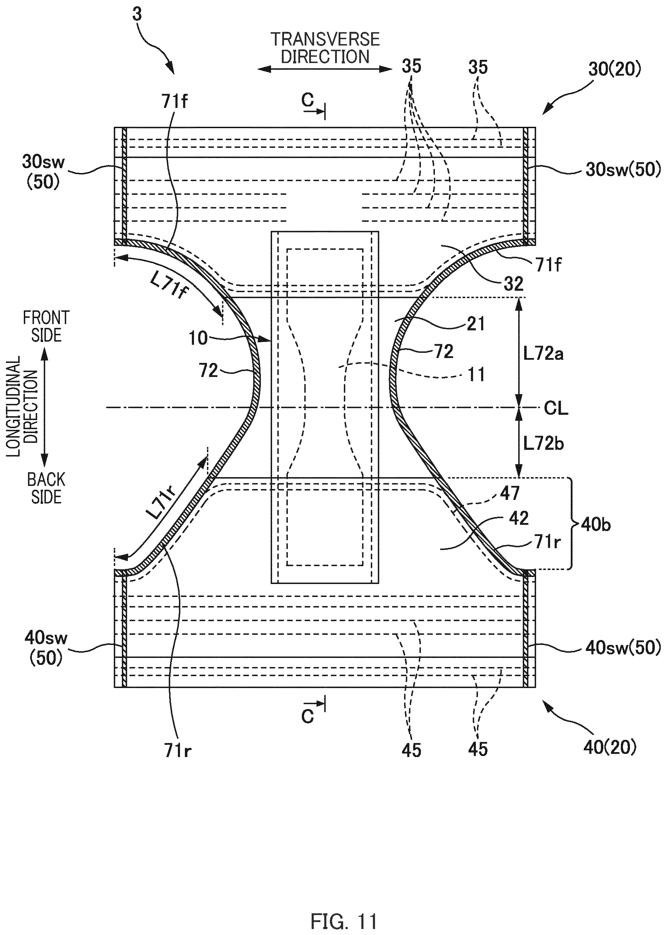

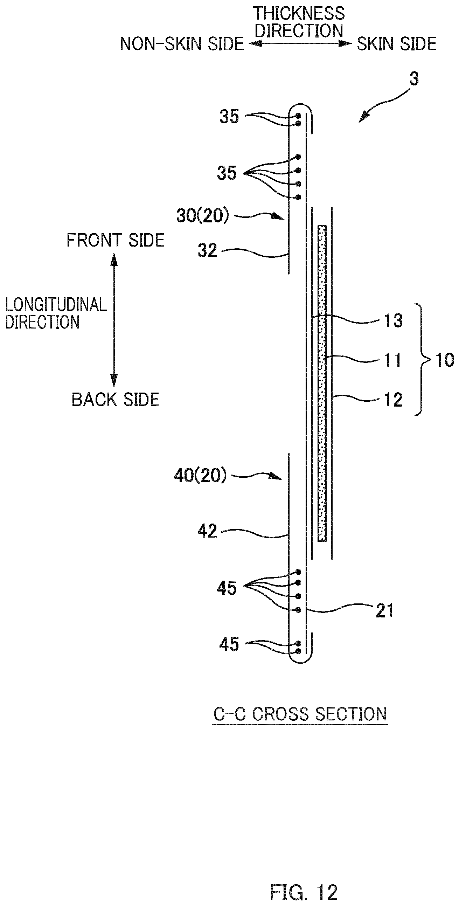

In a third embodiment, an underpants-shaped diaper 3 (hereinafter, also referred to as a "diaper 3") having a configuration partially different from those of the first embodiment and the second embodiment will be described. FIG. 11 is a plan view of the diaper 3 in the unfolded and stretched state. FIG. 12 is a schematic cross-sectional view taken along a line C-C in FIG. 11. It should be noted that the directions (e.g., the longitudinal direction, the transverse direction, or the like) in FIGS. 11A and 12 is the same as the directions in the first embodiment.

The diaper 3 of the third embodiment has the liquid-absorbent main body 10 and the waist member 20 that is joined to the non-skin side of the absorbent main body 10. However, in the diaper 3, as shown in FIG. 12, the skin-side sheet 21 that constitutes the front waist portion 30 (front skin-side sheet) and the skin-side sheet 21 that constitutes the back waist portion (back skin-side sheet) 40 are configured as an integral sheet member that continues in the longitudinal direction. On the other hand, the non-skin-side sheet 32 that constitutes the front waist portion 30 (front non-skin-side sheet) and the non-skin-side sheet 42 that constitutes the back waist portion 40 (back non-skin-side sheet) are configured as different sheet members that are non-continuous in the longitudinal direction. Hereinafter, a diaper having such a structure will also be referred to as a simple three-piece type disposable diaper.

When (back non-skin-side sheet) the diaper 3 in the unfolded state in FIG. 12A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, similar to the diaper in FIG. 1, the diaper 3 is in an underpants-shaped state having a waist opening BH and a pair of leg openings LH and LH.

In the diaper 3, the basic configuration and function of the absorbent main body 10 are substantially the same as those of the absorbent main body 10 of the diaper 1 of the first embodiment, and therefore description thereof is omitted.

Further, in the diaper 3, a portion of the waist member 20 located on the front side with respect to the central position CL in the longitudinal direction (the lengthwise direction of the absorbent main body 10) serves as the front waist portion 30, and a portion located on the back side with respect to the central position CL serves as the back waist portion 40. The front waist portion 30 includes: the single skin-side sheet 21 that is located on the skin side in the thickness direction and extends from one end side (front side) to the other end side (back side) in the longitudinal direction; the non-skin-side sheet 32 that is located on the front side in the longitudinal direction and is overlaid to be adjacent to the non-skin side of the skin-side sheet 21; and the waist elastic members 35 that are arranged between the skin-side sheet 21 and the non-skin-side sheet 32 in the thickness direction. The back waist portion 40 includes: the skin-side sheet 21 that is common to the front waist portion 30; the non-skin-side sheet 42 that is located on the back side in the longitudinal direction and is overlaid to be adjacent to the non-skin side of the skin-side sheet 21; and the waist elastic members 45 that are arranged between the skin-side sheet 21 and the non-skin-side sheet 42 in the thickness direction.

The skin-side sheet 21 is a hydrophobic nonwoven fabric sheet similar to the skin-side sheets 31 and 41 of the diaper 1. The non-skin-side sheets 32 and 42 are hydrophilic nonwoven fabric sheets similar to the non-skin-side sheets 32 and 42 of the diaper 1. That is, in the diaper 3, the non-skin-side sheets 32 and 42 are nonwoven fabric sheets having higher hydrophilicity than the skin-side sheet 21. Then, similar to the diaper 1, the waist elastic members 35 and 45 are formed of elastic strings or the like, and are attached between the skin-side sheet 21 and the non-skin-side sheets 32 and 42 in a state of being stretched in the lateral direction.

In the diaper 3 of the third embodiment, the leg openings LH are formed by the waist member 20. Further, in the region along the leg opening LH indicated by the hatched portions in FIG. 11, a region formed only by the skin-side sheet 21 of the waist member 20 is defined as the crotch leg-circumferential region 72 (second region 72), and regions formed by portions where the skin-side sheet 21 is overlaid on the non-skin-side sheets 32 and 42 are defined as the front leg-circumferential region 71f and the back leg-circumferential region 71r (first region 71).

As shown in FIGS. 11 and 12, in the diaper 3, in at least a region in the waist member 20 that overlap the pair of side joining portions 50 with respect to the vertical direction, a hydrophobic region formed of a hydrophobic nonwoven fabric (skin-side sheet 21) is provided in a surface located farthest on the skin side, and a hydrophilic region formed of a hydrophilic nonwoven fabric (non-skin-side sheets 32 and 42) is provided in a surface located farthest on the non-skin side. Therefore, the diaper 3 has a structure in which moisture is likely to be absorb from the wearer's skin side and evaporated into the atmosphere on the non-skin side.

In the diaper 3, the crotch leg-circumferential region 72 (second region 72) is formed of one sheet of hydrophobic nonwoven fabric (skin-side sheet 21). That is, in the leg openings LH, the hydrophobic nonwoven fabric is arranged farthest on the non-skin side of the second regions 72. This makes at least the non-skin side of a portion located at the lowermost end of the leg opening LH that comes into contact with the wearer's crotch portion (at the central position CL in the longitudinal direction) be a hydrophobic region, and the region has a structure in which moisture is less likely to be held. Accordingly, while the diaper 3 is put on, the wearer's skin is less likely to become wet in the crotch portion around the wearer's legs.

That is, in an underpants-shaped diaper having a so-called simple three-piece structure like the diaper 3, by using the hydrophobic nonwoven fabric for the portion that comes into contact with the wearer's crotch portion, the wearer can less likely to feel discomfort around the legs.

Further, in the leg opening LH of the simple three-piece type diaper 3, in the first region 71 in which the non-skin-side sheets 32 and 42 are arranged, the non-skin side is a hydrophilic region, and in the second region 72 formed of the skin-side sheet 21, the non-skin side is a hydrophobic region. In such a case, in the leg opening LH, moisture is likely to be evaporated in the first region 71 moisture is less likely to hold in the second region 72 that comes into contact with the wearer's crotch portion, making the wearer's crotch portion less likely to become wet. Accordingly, the wearer can be less likely to feel discomfort in a wide range of the crotch portion.

In the leg opening LH of the diaper 3, the sheet member arranged on the non-skin side with respect to the liquid-impermeable sheet 13a of the absorbent main body 10 is only the skin-side sheet 21. That is, in the second region 72 of the diaper 3, only one sheet of hydrophobic nonwoven fabric is arranged on the non-skin side with respect to the absorbent main body 10. By reducing the number of sheet members arranged in the second region 72 as small as possible and decreasing the stiffness of the region, while the diaper 3 is put on, the texture of the crotch portions of the leg openings LH against the skin is soft, and this allows the wearer to feel comfortable.

Further, as shown in FIG. 11, it is preferable that the length L72a of the second region 72 (the length of the portion along the second region 72) on the front side with respect to the central position CL in the longitudinal direction (the lengthwise direction of the absorbent main body 10) is longer than the length L72b of the second region 72 (the length of the portion along the second region 72) on the back side with respect to the central position CL (L72a > L72b). While the diaper 3 is put on, there are many opportunities to move the wearer's legs forward, such as when the wearer walks. Therefore, by making elongated the length L72a of the second region 72 on the front side with respect to the central position CL, the hydrophobic region on the front side of the leg opening LH becomes wider, making the legs less likely to become wet even in the case where the wearer moves the legs forward. Accordingly, the wearer can be less likely to feel discomfort while the diaper is put on.

Further, it is preferable that in the first region 71 that forms the leg opening LH, the length L71r of the back leg-circumferential region 71r (the length of the portion along the region 71r) on the back side with respect to the central position CL in the longitudinal direction is longer than the length L71f of the front leg-circumferential region 71f (the length of the portion along the region 71f) on the front side with respect to the central position CL (L71r > L71f). The back leg-circumferential region 71r is a region that is likely to come into wide contact with the wearer's buttocks and that is likely to be pressed against the wearer's body due to the body pressure which is to be applied in a case such that the wearer sits down in a state where the wearer puts on the diaper **1.** Therefore, by making elongated the length L71r of the back leg-circumferential region 71r, the hydrophobic region on the back side of the leg opening LH (buttocks side), and the buttocks are less likely to become wet even in the case where the wearer takes a seated posture. Accordingly, the wearer can be less likely to feel discomfort while the diaper is put on.

Further, it is preferable that the waist member 20 (30, 40) of the diaper 3 has a two-layer structure in which two sheet members are overlaid in the thickness direction, namely a single skin-side sheet 21 formed of hydrophobic nonwoven fabric and a single non-skin-side sheet 32 (42) formed of hydrophilic nonwoven fabric. Not providing extra sheet member in the waist member 20 makes it less likely to inhibit the absorption and evaporation of moisture, and this makes it possible to more efficiently evaporate the moisture. Further, the hydrophilic region provided on the non-skin-side surface of the waist member 20 is formed of one sheet of hydrophilic nonwoven fabric, and therefore the hydrophilic region is formed in a wide range of the waist member 20, which makes it possible to further enhance evaporation property.

Further, the back waist portion 40 of the diaper 3 has the buttocks cover 40b below the side joining portions 50 in the vertical direction (see FIG. 11). Moisture such as sweat can be likely to be evaporated by the buttocks cover 40b in the wearer's buttocks. Further, the buttocks cover 40b has the curved elastic members 47 extending along the end edge portions of the buttocks cover 40b (back leg-circumferential region 71r). The stretchability of the curved elastic members 47 makes the buttocks cover 40b likely to fit to the wearer's buttocks while the diaper 3 is put on. Further, since stretchability acts along the back leg-circumferential region 71r, the back leg-circumferential region 71r is less likely to turn up, enabling to make the wearer less likely to feel unpleasantness or discomfort and to make it likely to exhibit the moisture evaporation function by the back waist portion 40.

### Modified example

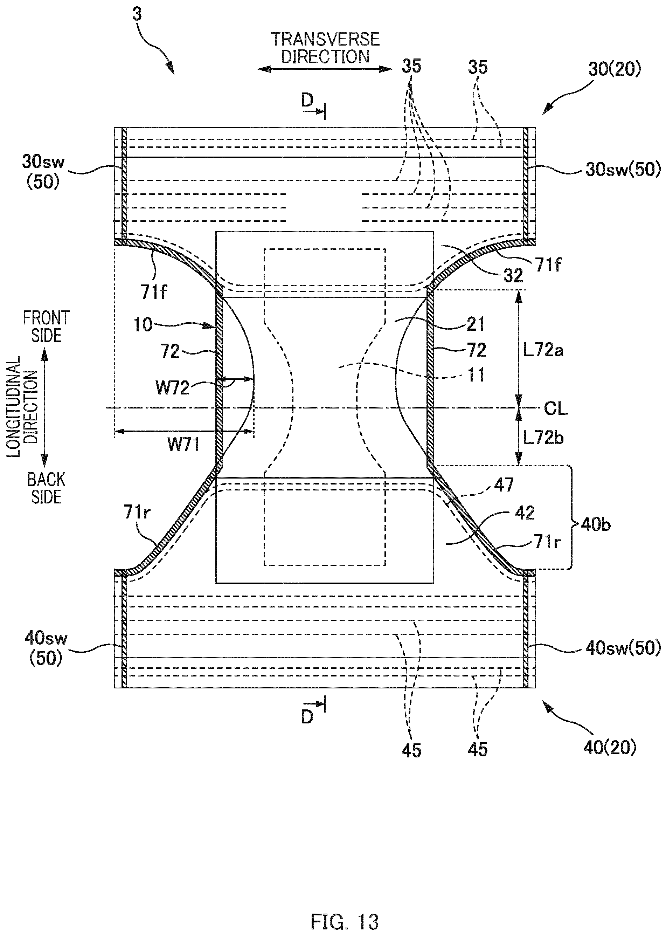

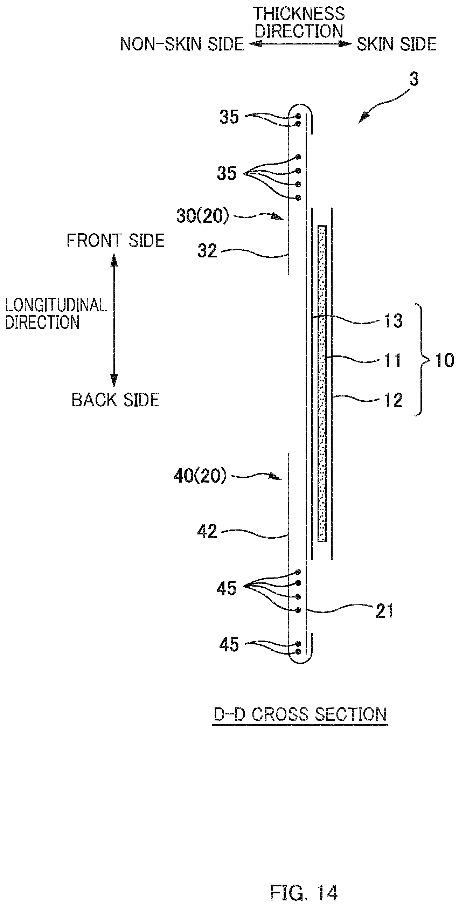

In the diaper 3, similar to diaper 2 of the second embodiment, the shape of the absorbent main body 10 may be deformed in a manner such that the lateral length (width) of the absorbent main body 10 is narrower than the minimum value of the lateral length (width) of the waist member 20. FIG. 13 is a plan view of a modified example of the diaper 3 in the unfolded and stretched state. FIG. 14 is a schematic cross-sectional view taken along a line D-D in FIG. 13.

In a modified example of the diaper 3, in the regions along the leg openings LH indicated by the hatched portions in FIG. 13, regions formed by the side edge portions of the absorbent main body 10 are the crotch leg-circumferential regions 72 (second region 72). On the other hand, the regions formed by the lower end edge portions of the front waist portion 30 are the front leg-circumferential regions 71f (first region 71), and the regions formed by the lower end edge portions of the back waist portion 40 are the back leg-circumferential regions 71r (first region 71). Other configurations are substantially the same as those of the diaper 3 described in FIG. 11.

In the diaper 3 of the modified example, the exterior sheet 13b of the absorbent main body 10 is formed of a hydrophobic nonwoven fabric. That is, in the leg openings LH, the hydrophobic nonwoven fabric is arranged farthest on the non-skin side of the second regions 72. This makes at least the non-skin side of a portion located at the lowermost end of the leg opening LH that comes into contact with the wearer's crotch portion (at the central position CL in the longitudinal direction) be a hydrophobic region, and the region has a structure in which moisture is less likely to be held. Accordingly, the wearer's skin is less likely to become wet in the wearer's crotch portion.

That is, even in the case where the diaper 3 has the configuration as shown in FIG. 13 (in a modified example), by using a hydrophobic nonwoven fabric as the sheet member of the outermost layer of the absorbent main body 10 (exterior sheet 13b) that comes into contact with the wearer's crotch portion, the wearer can be less likely to feel discomfort around the legs.

Further, similar to the diaper 2 described above, it is desirable that in the lateral direction of the diaper 3 of the modified example, the distance W72 between the outermost end of the absorbent main body 10 and the innermost end of the waist member 20 is smaller than 1/2 of the distance W71 between the outermost end of the waist member 20 and the innermost end of the waist member 20. Accordingly, the side edge portions of the absorbent main body 10 (crotch leg-circumferential regions 72) are prevented from excessively extending outward in the lateral direction, and while the diaper 3 is put on, the wearer easily moves the legs and can make the wearer less likely to feel discomfort around the legs.

### Fourth Embodiment

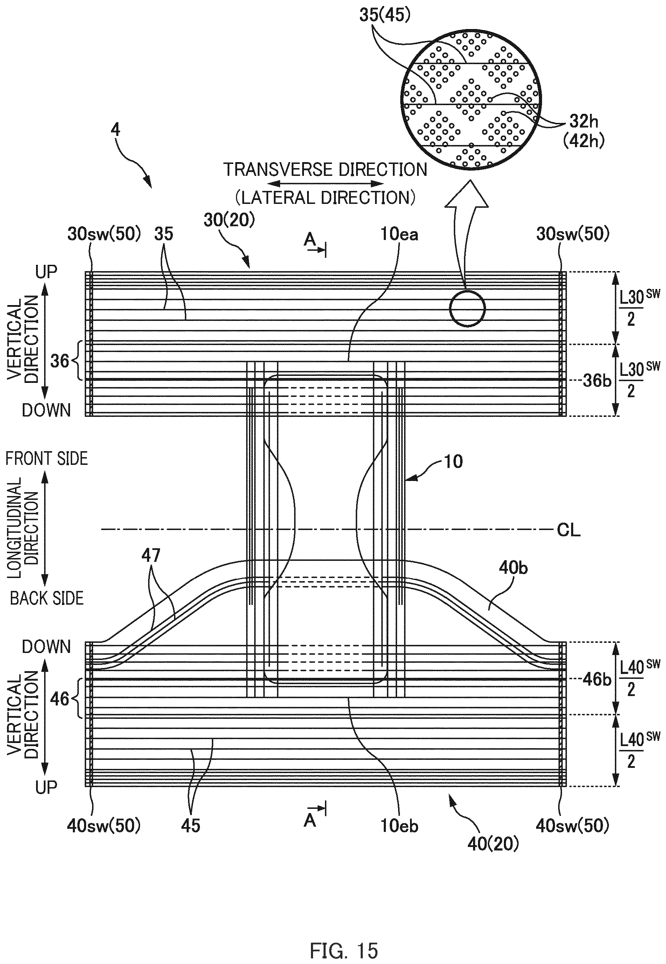

The following describes an underpants-shaped absorbent article according to a fourth embodiment by way of example of an underpants-shaped disposable diaper (hereinafter, also referred to as a "diaper 4"). FIG. 15 is a plan view of the diaper 4 in the unfolded and stretched state. As shown in FIG. 15, the basic configuration of the diaper 4 is substantially the same as that of the diaper 1 of the first embodiment (see FIGS. 2, 1, 3, and 4). Therefore, a detailed description of the entire configuration of the diaper 4 is omitted.

### Side Joining Portion 50

In the diaper 4, a hydrophilic nonwoven fabric containing a hydrophilic oil agent is used for a part of the waist member 20 (30, 40), enhancing the water absorbency. On the other hand, there is a risk that the following problems occur in the side joining portions 50 due to the influence of the hydrophilic oil agent contained in the nonwoven fabric.

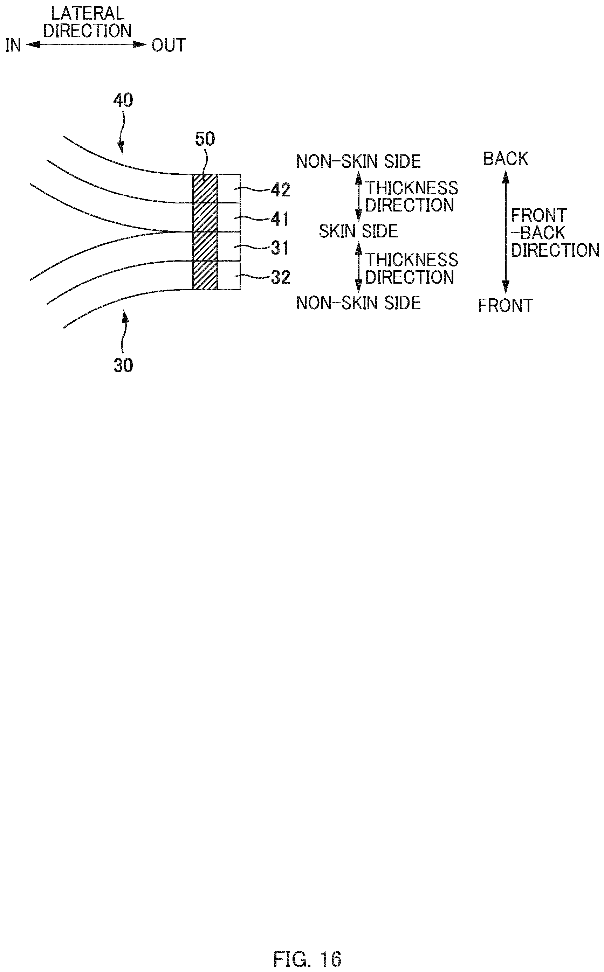

FIG. 16 is a schematic cross-sectional view of the side joining portion 50 that joins the front waist portion 30 and the back waist portion 40, when viewed in the vertical direction. As shown in FIG. 16, at the lateral outer end portions of the waist member 20, the front waist portion 30 and the back waist portion 40 are joined in a state of being overlaid on each other in the thickness direction, forming the side joining portions 50. In each side joining portion 50, the skin-side sheet 31 of the front waist portion 30 and the skin-side sheet 41 of the back waist portion 40 are arranged so as to face each other in the thickness direction, and are joined.

In the present embodiment, the side joining portions 50 of the diaper 4 are joined by using welding means such as ultrasonic welding, and therefore there is a risk of causing a problem if the surfaces that face each other in the thickness direction in the side joining portions 50 are each formed of a hydrophilic nonwoven fabric. Specifically, in the case where both the skin-side sheet 31 of the front waist portion 30 and the skin-side sheet 41 of the back waist portion 40 are hydrophilic sheet portions containing a hydrophilic oil agent, there is a risk that a coating formed by the hydrophilic oil agent makes insufficient the welding strength of the ultrasonic welding.

In such a case, there is a case where the joinings of the side joining portions 50 are peeled off while the diaper 4 is put on, and the diaper 4 cannot maintain the underpants shape as shown in FIG. 1. For example, when the wearer puts on the underpants-shaped diaper 4, an operation of passing the legs through the leg openings LH of the diaper 4 and pulling up the diaper 4 from the toe side to the crotch side is performed. At this time, stress is likely to be concentrated at the lower end portion of the side joining portion 50 (the upper end portion of the leg opening LH), and therefore if the joining strength of the side joining portion 50 is insufficient, there is a risk that the entire side joining portion 50 is peeled off by starting to pee off from the lower end portion.

In contrast, in the present embodiment, the skin-side sheet 31 and the skin-side sheet 41 that face each other in the thickness direction in the side joining portions 50 are both formed of a hydrophobic nonwoven fabric not containing a hydrophilic oil agent. That is, the farthest-on-skin-side surfaces of the front waist portion 30 and the back waist portion 40 each have a hydrophobic sheet portion having a low hydrophilic oil agent content per unit volume. Therefore, the joining strength of the side joining portion 50 is less likely to decrease compared with the case where hydrophilic sheet portions having a high hydrophilic oil agent content are arranged facing each other. This suppresses the peeling of the lower end portion of the side joining portion 50 at the time of putting on the diaper 4, enabling to make the underpants shape likely to maintain.

It should be noted that, in the case where the hydrophobic sheet portion is provided on at least one of the skin-side sheet 31 and the skin-side sheet 41 that face each other in the thickness direction, the influence of the oil film of the hydrophilic oil agent formed on the facing surfaces of the side joining portions 50 is reduced, and this makes it possible to easily suppress a decrease in the joining strength. In the present embodiment, as shown in FIG. 16, both the skin-side sheet 31 and the skin-side sheet 41 which are arranged farthest on the skin side are hydrophobic nonwoven fabrics. In other words, in the lower end portion of the side joining portion 50, the hydrophobic sheet portion is provided on both of the farthest-on-skin-side surface of the front waist portion 30 and the farthest-on-skin-side surface of the back waist portion 40. Therefore, the influence of the hydrophilic oil agent in the lower end portion of the side joining portion 50 is further reduced, and this can make it more easily to suppress a decrease in the joining strength.

The content of the hydrophilic oil agent per unit volume in each sheet member can be measured as follows. First, a hydrophilic oil agent is extracted from the sheet member. Approximately 5 g of a to-be-measured sheet member is cut out to obtain a sample, and the oven dry mass is obtained. The sample is placed in a Soxhlet extractor specified in JIS R 3503, without using a cylindrical filter paper. Then, 100 mL to 150 mL of a liquid mixture of ethanol (G.R.) of JIS K 8102 and benzene (G.R.) of JIS K 8858 (volume ratio 1:2) (hereinafter, referred to as an ethanol-benzene liquid mixture) is placed into the attached flask. However, when the recovered ethanol-benzene liquid mixture is used, the mixture is prepared in a manner such that the moisture content is (1.7 ± 0.5)%, and used. Then, the solution is placed on an aqueous bath and heated for 3 hours to an extent that the extract maintains a weak boiling state. Then, the solution accumulated in the sample portion is returned to the flask, the content of the flask is concentrated to 5 mL (if necessary, filtrated with a 1G1 or 3G1 glass filter), and then the flask is transferred to a weighing bottle whose constant weight was determined at 105 ± 2°C in advance. The extraction flask is washed with the ethanol-benzene liquid mixture at about 40°C, the washing liquid (in a case of using a glass filter, after filtration through the glass filter) is combined in the weighing bottle, and thereafter the solvent is volatilized on the aqueous bath. Then, the resultant is left to stand for 1.5 hours in a thermostatic dryer at 105 ± 2°C, and cooled in a desiccator, and the mass is weighed. The extracted content is expressed as a percentage to the oven dry sample mass of the extracted amount of the ethanol-benzene liquid mixture, and the average value of two times of measurement is rounded to two decimal places according to Rule B of JIS Z 8401 (Rules for Rounding-off). The value obtained by dividing the mass of the oil agent extracted in this manner by the volume of the sample is defined as the content of the hydrophilic oil agent per unit volume in the sheet member.

Further, the hydrophobic skin surface sheets 36 and 46 are respectively provided on the skin side in the lower end portions of the side joining portions 50 of the diaper 4. In FIGS. 15 and 3, the skin surface sheet 36 is provided farthest on the skin side between the central position and the lower end position of the side portion 30sw (50) of the front waist portion 30 in the vertical direction, and in at least a part of the region, two hydrophobic sheet portions are provided overlapping each other. Similarly, farthest on the skin side between the central position and the lower end position of the side portion 40sw (50) of the back waist portion 40 in the vertical direction, two hydrophobic sheet portions are provided overlapping each other. It should be noted that FIG. 3 shows a central cross-sectional view (A-A cross section) in the lateral direction, and therefore, the absorbent main body 10 is arranged between the skin surface sheet 36 (46) and the skin-side sheet 31 (41).

By providing two hydrophobic sheet portions overlapping each other in the thickness direction on the skin-side surface of the waist member 20, the influence of the hydrophilic oil agent contained in the hydrophilic sheet portion arranged on the non-skin side is reduced in the side joining portion 50, making it more likely to suppress a decrease in the joining strength. It should be noted that the hydrophobic sheet portion composed of three or more layers may be provided overlapping each other on the skin-side surface of the waist member 20. In such a case, the influence of the hydrophilic sheet portion located on the non-skin side can be further reduced.

Further, as shown in FIG. 15, the lower end 36b (46b) of the skin surface sheet 36 (46) is positioned between the central position and the lower end position of the front waist portion 30 (40) in the vertical direction. As described above, the influence of the hydrophilic oil agent is reduced in the region in which the skin surface sheet 36 is provided, and this makes it possible to make the joining strength of the side joining portion 50 stronger than in other regions. Therefore, even in the case where peeling occurs at the lower end portion of the side joining portion 50 while the diaper 4 is put on and the peeling propagates toward the upper side in the vertical direction along the side joining portion 50, the propagation of the peeling can be stopped at the lower end position 36b (46b) of the skin surface sheet 36 (46). That is, the skin surface sheet 36 functions as a rip stopper of the side joining portion 50, making it possible to suppress the peeling of the entire side joining portion 50. Accordingly, the underpants shape of the diaper 4 is more likely to be maintained.

Further, in the waist member 20 (30, 40) of the diaper 4, in the entire region from the upper end to the lower end of the side joining portion 50 in the vertical direction, the hydrophobic sheet portion (skin-side sheets 31 and 41) may be provided farthest on the skin side. According to the above-described configuration, a decrease in the joining strength of the side joining portion 50 as a whole is likely to be suppressed, and the underpants shape of the diaper 4 can be likely to be stably maintained.

On the other hand, in the diaper 4, as shown in FIG. 3, the folded-back portion 32f (42f) in which the non-skin-side sheet 32 (42) is folded back toward the skin side may be provided at the upper end portion of the front waist portion 30 (40) in the vertical direction. In this case, in the upper end portion of the side joining portion 50 in the vertical direction, the hydrophilic sheet portion is provided farthest on the skin side. That is, in the upper end portion of the side joining portion 50 in the vertical direction, the amount of the hydrophilic oil agent contained per unit volume is large compared with the lower end portion. Therefore, the joining strength of the upper end portion of the side joining portion 50 of the diaper 4 is lower than that of the lower end portion.

As described above, since a load is likely to be applied to the upper end portion of the side joining portion 50 when putting on the diaper 4, it is important to make the joining portion less likely to be peeled off. On the other hand, when removing the diaper 4 from the wearer's body after the diaper 4 is used, an operation is performed of grabbing the vicinity of the upper end portions of the side joining portions 50, pulling the diaper 4 in the front-back direction, separating the front waist portion 30 and the back waist portion 40, which are connected by the side joining portions 50. That is, an operation of tearing the side joining portion 50 from the upper end portion side to the lower side is performed. Therefore, it is desirable that the joining strength is set to be weak to a certain extent at the upper end portion of the side joining portion 50.

In particular, in FIG. 3, the hydrophilic folded-back portion 32f (42f) is arranged farthest on the skin side in the upper end portion of the side joining portion 50. That is, the hydrophilic sheet portion is provided farthest on the skin side in the upper end portion of the side joining portion 50. Therefore, in the upper end portion, the hydrophilic sheet portions face each other, making it possible to further weaken the joining strength of the side joining portion 50.

However, it is not necessary that the folded-back portion 32f (42f) is exposed, farthest on the skin side in the thickness direction, in the upper end portion of the side joining portion 50. That is, the folded-back portion 32f (42f) may be folded back toward the non-skin side with respect to the skin-side sheet 31 (41). Also in this case, since the amount of the hydrophilic oil agent per unit volume in the upper end portion of the side joining portion 50 is large, the above-described effect can be obtained. Further, in the upper end portion of the side joining portion 50, a hydrophilic sheet member (hydrophilic sheet portion) different from the folded-back portion 32f (42f) may be separately provided.

As described above, in the diaper 4 of the present embodiment, the content of the hydrophilic oil agent in the upper end portion of the side joining portion 50 is high, and therefore the operation of tearing the side joining portion 50 from the upper end portion side can be easily performed. That is, the side joining portion 50 of the diaper 4 is less likely to be peeled off from the lower end portion side and is easily peeled off from the upper end portion side. Accordingly, the user can perform an operation of putting on and taking off the diaper 4 without any stress.

Further, since the hydrophilic sheet portion composed of two or more layers overlaid in the thickness direction are provided in the upper end portion of the side joining portion 50, it is possible to increase the strength of the waist member 20 itself in the region. Therefore, when removing the diaper 4, even if the upper end portion of the side joining portion 50 is grabbed, pulling the front waist portion 30 and the back waist portion 40 in the front-back direction, it makes less likely to tear the sheet members that constitute the front waist portion 30 and the back waist portion 40. That is, a force that pulls the waist member 20 in the front-back direction does not act to tear the fabric of the waist member 20, but becomes more likely to act to peel off the side joining portion 50. Further, since the amount of the hydrophilic oil agent per unit volume is large in the portion in which the hydrophilic sheet portion is composed of two or more layers overlaid, it is possible to decrease the joining strength of the side joining portion 50 as described above. This makes it easier to perform the operation of peeling off the side joining portion 50 from the upper end portion side to the lower side.

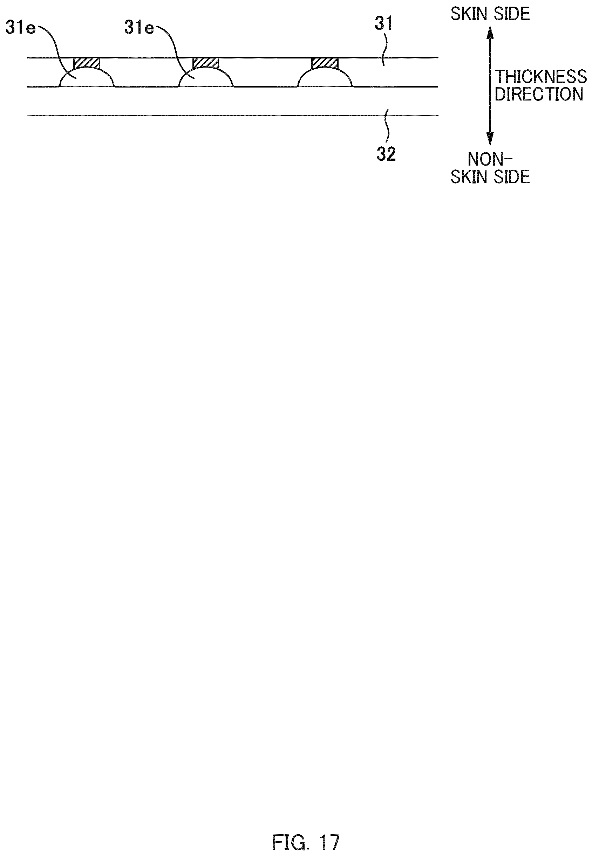

Further, compressed portions may be provided in the skin-side sheets 31 and 41 that constitute the waist member 20 (30, 40). FIG. 17 is a schematic cross-sectional view showing a cross-sectional shape of the front waist portion 30 in the thickness direction.

In FIG. 17, a plurality of compressed portions 31e in which the skin-side sheet 31 is compressed from the non-skin side to the skin side in the thickness direction are provided on the skin-side sheet 31 (hydrophobic sheet portion). By providing the plurality of compressed portions 31e, 31e .... on the surface of the skin-side sheet 31, it increases the strength of the skin-side sheet 31, enabling to make the sheet less likely to tear. In the compressed portion 31e, compressed bottom portions (indicated by the hatched portions in FIG. 17) are arranged at positions spaced apart from the non-skin-side sheet 32 (hydrophilic sheet portion) by a predetermined distance in the thickness direction. Therefore, the compressed portions 31e are less likely to be affected by the hydrophilic oil agent contained in the non-skin-side sheet 32 (hydrophilic sheet portion), enabling to make it more likely to suppress a decrease in the joining strength of the side joining portion 50. Accordingly, the side joining portion 50 can be prevented from being peeled off from the lower end portion.

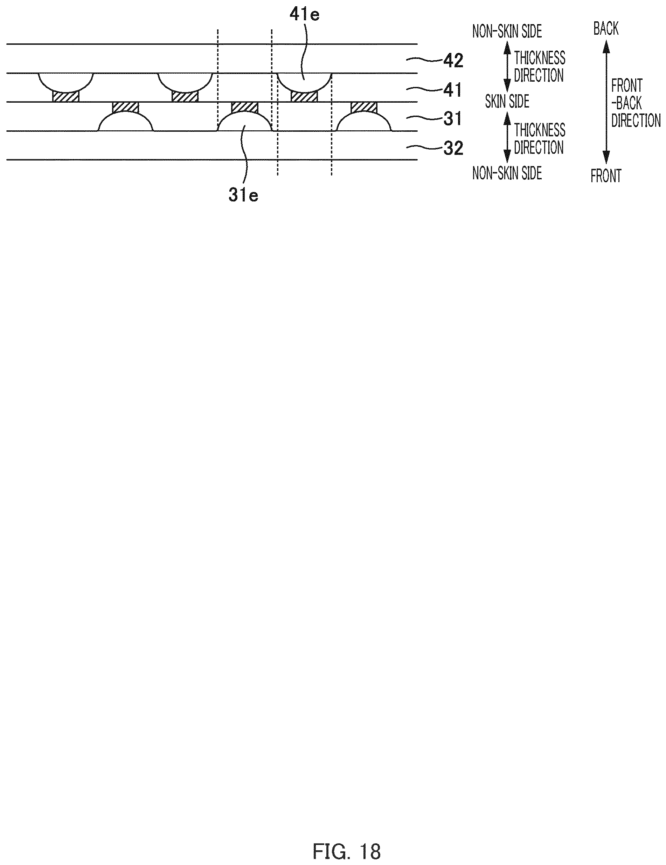

Further, it is desirable that the side joining portion 50 has a portion where the position of the compressed portion 31e provided in the front waist portion 30 and the position of a compressed portion 41e provided in the back waist portion 40 do not overlap each other when viewed in the thickness direction. FIG. 18 is a schematic cross-sectional view showing the relationship between the positions of the compressed portions 31e of the front waist portion 30 and the positions of the compressed portions 41e of the back waist portion 40 in the side joining portion 50.

In FIG. 18, the positions of the compressed portions 31e and the positions of the compressed portions 41e are arranged to be spaced apart from each other when viewed in the thickness direction. That is, the bottom portions (portions indicated by the hatched portions in FIG. 18) of the compressed portions 31e and 41e having high stiffness are arranged at positions displaced from each other. Therefore, the stiffness in the side joining portion 50 is prevented from becoming excessively high, and the texture of the diaper 4 while the diaper 4 is put on is less likely to deteriorate. Further, in the case where the bottom portions of the compressed portions 31e and 41e having high stiffness overlap each other, there is a possibility that welding defects are generated when the side joining portion 50 is formed. In contrast, the positional deviation between the bottom portions of the compressed portions 31e and 41e can make welding defects or the like less likely to occur. Therefore, the peeling of the lower end portion of the side joining portion 50 can be easily suppressed.

### Fifth Embodiment

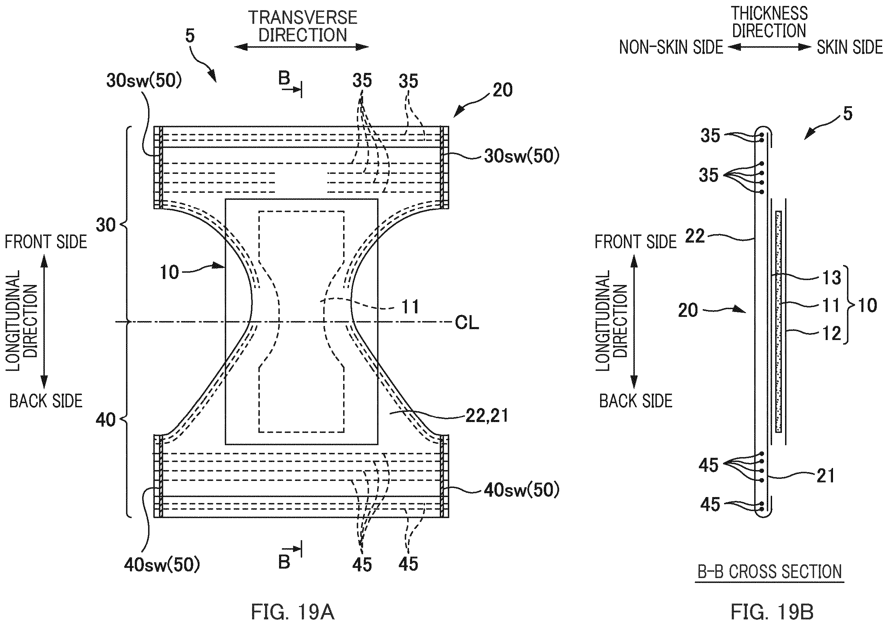

In a fifth embodiment, an underpants-shaped diaper 5 (hereinafter, also referred to as a "diaper 5") having a configuration partially different from that of the fourth embodiment will be described. FIG. 19A is a plan view of the diaper 5 in the unfolded and stretched state. FIG. 19B is a schematic cross-sectional view taken along a line B-B in FIG. 19A. It should be noted that the directions (e.g., the longitudinal direction, the transverse direction, or the like) in FIGS. 19A and 19B is the same as the directions in the fourth embodiment.

The diaper 5 of the fifth embodiment has the liquid-absorbent absorbent main body 10, and the waist member 20 that is arranged on the non-skin side of the absorbent main body 10. However, in the diaper 5, the front waist portion 30 and the back waist portion 40 are integrally configured. That is, the diaper 5 is a so-called two-piece type disposable diaper formed of the absorbent main body 10 serving as an interior body and the waist member 20 serving as an exterior body.

When shaping the diaper 5 in the unfolded state in FIG. 19A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, as in FIG. 1, the diaper 5 is in an underpants-shaped state having a waist opening BH and a pair of leg openings LH and LH.

In the diaper 5, the basic configuration and function of the absorbent main body 10 are substantially the same as those of the absorbent main body 10 of the diaper 4 of the fourth embodiment, and therefore description thereof is omitted. It should be noted that, in FIG. 19A, the crotch portion including the central position CL in the longitudinal direction (the lengthwise direction of the absorbent main body 10) has a portion in which the lateral width of the absorbent main body 10 is wider than the lateral width of the waist member 20. Therefore, during usage of the diaper 5, in the region in the vicinity of the wearer's crotch portion, two lateral end edge portions (edges) of the absorbent main body 10 come into contact with the wearer's legs.

Further, in the diaper 5, a portion of the waist member 20 located on the front side with respect to the central position CL in the longitudinal direction (the lengthwise direction of the absorbent main body 10) serves as the front waist portion 30, and a portion located on the back side with respect to the central position CL serves as the back waist portion 40. The waist member 20 includes: a skin-side sheet 21; a non-skin-side sheet 22 that is overlaid to be adjacent to the non-skin side of the skin-side sheet 21; and waist elastic members 35 and 45 that are arranged between the skin-side sheet 21 and the non-skin-side sheet 22 in the thickness direction (see FIG. 19B).

The skin-side sheet 21 is a hydrophobic nonwoven fabric sheet similar to the skin-side sheets 31 of the diaper 4. The non-skin-side sheet 22 is a hydrophilic nonwoven fabric similar to the non-skin-side sheets 32 of the diaper 4. That is, the non-skin-side sheet 22 is formed of nonwoven fabric having higher hydrophilicity than the skin-side sheet 21. Then, similar to the diaper 4, the waist elastic members 35 and 45 are formed of elastic strings or the like, and are attached between the skin-side sheet 21 and the non-skin-side sheet 22 in a state of being stretched in the lateral direction.

In the diaper 5 of the fifth embodiment, similar to the diaper 4 of the fourth embodiment, it also can make it likely to suppress the peeling of the lower end portions of the side joining portions 50. That is, the hydrophobic sheet portion having a low hydrophilic oil agent content per unit volume is provided on the farthest-on-skin-side surfaces of the front waist portion 30 and the back waist portion 40, in the lower end portion of the side joining portion 50, and therefore the joining strength of the side joining portion 50 is less likely to decrease compared with the case where the hydrophilic sheet portions are arranged facing each other. Accordingly, it suppresses the peeling of the lower end portion of the side joining portion 50 at the time of putting on the diaper 5, and the underpants shape can make it likely to maintain.

### Sixth Embodiment

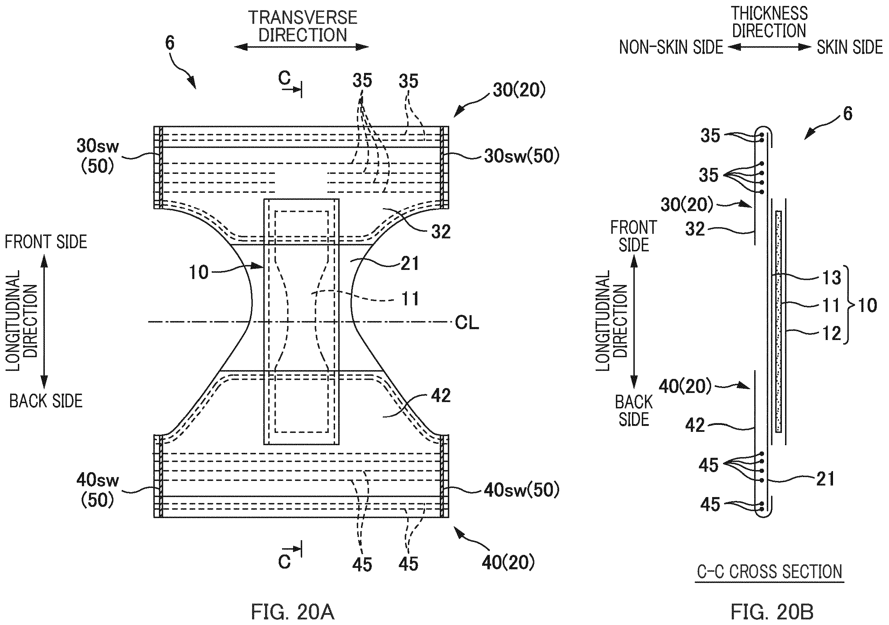

In a sixth embodiment, an underpants-shaped diaper 6 (hereinafter, also referred to as a "diaper 6") having a configuration partially different from those of the fourth embodiment and the fifth embodiment will be described. FIG. 20A is a plan view of the diaper 6 in the unfolded and stretched state. FIG. 20B is a schematic cross-sectional view taken along a line C-C in FIG. 20A. It should be noted that the directions (e.g., the longitudinal direction, the transverse direction, or the like) in FIGS. 20A and 20B is the same as the directions in the fourth embodiment.

The diaper 6 of the sixth embodiment has the liquid-absorbent absorbent main body 10, and the waist member 20 that is arranged on the non-skin side of the absorbent main body 10. However, in the diaper 6, as shown in FIG. 20B, a part of the member that constitutes the front waist portion 30 (skin-side sheet 21) and a part of the member that constitutes the back waist portion 40 (skin-side sheet 21) are integrally configured. Hereinafter, a diaper having such a structure will also be referred to as a simple three-piece type disposable diaper.

When shaping the diaper 6 in the unfolded state in FIG. 20A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, as in FIG. 1, the diaper 6 is in an underpants-shaped state having a waist opening BH and a pair of leg openings LH and LH.

In the diaper 6, the basic configuration and function of the absorbent main body 10 are substantially the same as those of the absorbent main body 10 of the diaper 4 of the fourth embodiment, and therefore description thereof is omitted.

Further, in the diaper 6, a portion of the waist member 20 located on the front side with respect to the central position CL in the longitudinal direction (the lengthwise direction of the absorbent main body 10) serves as the front waist portion 30, and a portion located on the back side with respect to the central position CL serves as the back waist portion 40. The front waist portion 30 includes: the single skin-side sheet 21 that is located on the skin side in the thickness direction and extends from the one end side (front side) to the other end side (back side) in the longitudinal direction; the non-skin-side sheet 32 that is located on the front side in the longitudinal direction and is overlaid to be adjacent to the non-skin side of the skin-side sheet 21; and the waist elastic members 35 that are arranged between the skin-side sheet 21 and the non-skin-side sheet 32 in the thickness direction. The back waist portion 40 includes: the skin-side sheet 21 that is common to the front waist portion 30; the non-skin-side sheet 42 that is located on the back side in the longitudinal direction and is overlaid to be adjacent to the non-skin side of the skin-side sheet 21; and the waist elastic members 45 that are arranged between the skin-side sheet 21 and the non-skin-side sheet 42 in the thickness direction.

The skin-side sheet 21 is a hydrophobic nonwoven fabric sheet similar to the skin-side sheets 31 of the diaper 4. The non-skin-side sheets 32 and 42 are hydrophilic nonwoven fabrics similar to the non-skin-side sheets 32 and 42 of the diaper 4. That is, the non-skin-side sheets 32 and 42 are each formed of a nonwoven fabric sheet having higher hydrophilicity than the skin-side sheet 21. Then, similar to the diaper 4, the waist elastic members 35 and 45 are formed of elastic strings or the like, and are attached between the skin-side sheet 21 and the non-skin-side sheets 32 and 42 in a state of being stretched in the lateral direction.

In the diaper 6 of the sixth embodiment, similar to the diaper 4 of the fourth embodiment, it also can make it likely to suppress the peeling of the lower end portions of the side joining portions 50.

That is, the hydrophobic sheet portion having a low hydrophilic oil agent content per unit volume is provided on the farthest-on-skin-side surfaces of the front waist portion 30 and the back waist portion 40, in the lower end portion of the side joining portion 50, and therefore the joining strength of the side joining portion 50 is less likely to decrease compared with the case where the hydrophilic sheet portions are arranged facing each other. Accordingly, it suppresses the peeling of the lower end portion of the side joining portion 50 at the time of putting on the diaper 6, and the underpants shape can make it likely to maintain.

### Other Embodiments

Although the above embodiments of the present invention have been described, but the above-described embodiments are intended to facilitate the understanding of the present invention and are not intended to limit the interpretation of the present invention. In addition, the present invention can be modified or improved within the scope of the gist of the present invention, and it is needless to say that equivalents thereof are included in the present invention.

### REFERENCE SIGNS LIST

1: diaper (underpants-shaped absorbent article) (first embodiment),
2: diaper (underpants-shaped absorbent article) (second embodiment),
3: diaper (underpants-shaped absorbent article) (third embodiment),
4: diaper (underpants-shaped absorbent article) (fourth embodiment),
5: diaper (underpants-shaped absorbent article) (fifth embodiment),
6: diaper (underpants-shaped absorbent article) (sixth embodiment),
10: absorbent main body,
11: absorbent core, 11b: core-wrapping sheet, 11c: narrow portion,
12: top sheet,
13: back sheet, 13a: liquid-impermeable sheet, 13b: exterior sheet,
15: leak-proof wall portion, 16: leak-proof-wall elastic member, 17: leg elastic member,
20: waist member,
21: skin-side sheet (hydrophobic nonwoven fabric),
22: non-skin-side sheet (hydrophilic nonwoven fabric),
30: front waist portion,
30sw: side portion,
31: skin-side sheet (hydrophobic nonwoven fabric),
31e: compressed portion,
32: non-skin-side sheet (hydrophilic nonwoven fabric), 32f: folded-back portion, 32h: hole portion,
35: waist elastic member,
36: skin surface sheet, 36b: lower end,
40: back waist portion, 40b: buttocks cover,
40sw: side portion,
41: skin-side sheet (hydrophobic nonwoven fabric),
41e: compressed portion,
42: non-skin-side sheet (hydrophilic nonwoven fabric), 42f: folded-back portion, 42h: hole portion,
45: waist elastic member,
46: skin surface sheet, 46b: lower end,
47: curved elastic member, 50: side joining portion,
60: welding portion,
60s: welding portion pair,
71: first region, 71f: front leg-circumferential region, 71r: back leg-circumferential region,
72: second region (crotch leg-circumferential region)
BH: waist opening, LH: leg opening,
CL: central position (longitudinal direction, lengthwise direction)

## Claims

1. An underpants-shaped absorbent article having a vertical direction, a lateral direction, and a front-back direction that intersect with each other,
the underpants-shaped absorbent article comprising:
a liquid-absorbent absorbent main body (10);
a waist member (20) in which a front waist portion (30) and a back waist portion (40) are annularly joined by a pair of side joining portions that is provided at two end portions in the lateral direction; and
a pair of leg openings (LH,LH)through which a wearer's legs are passed,
in the waist member, at least in a part of a region that overlap the pair of side joining portions with respect to the vertical direction, a hydrophilic region being provided farthest on a non-skin side,
in regions extending along the leg openings, at least in a portion that are located at a lowermost end, a hydrophobic region being provided farthest on the non-skin side,
the hydrophobic region having a lower hydrophilicity than the hydrophilic region, wherein;
in an unfolded state,
the front waist portion is joined to a one side of the absorbent main body in a longitudinal direction, and
the back waist portion is joined to another side in the longitudinal direction,
a hydrophilic nonwoven fabric is provided farthest on the non-skin side in at least one of the front waist portion and the back waist portion, and
a hydrophobic nonwoven fabric is provided farthest on a non-skin side in the absorbent main body,
the hydrophobic nonwoven fabric having a lower hydrophilicity than the hydrophilic nonwoven fabric, and **characterized in that**;
the leg opening has a first region (71) and a second region (72) on and below a lower end of the side joining portion,
the first region being constituted by the waist member,
the second region being constituted by the absorbent main body,
a portion of the first region farthest on the non-skin side is a hydrophilic region, and
a portion of the second region farthest on the non-skin side is a hydrophobic region.

2. The underpants-shaped absorbent article according to claim 1, wherein
in an unfolded state,
the waist member is integrally constituted such that the front waist portion and the back waist portion are continuous in a longitudinal direction,
the waist member is joined to a non-skin side of the absorbent main body,
a hydrophilic nonwoven fabric is provided farthest on a non-skin side of the waist member,
a hydrophobic nonwoven fabric is provided farthest on a non-skin side of the absorbent main body,
the hydrophobic nonwoven fabric having a lower hydrophilicity than the hydrophilic nonwoven fabric, and
a minimum value of a lateral width of the waist member is smaller than a minimum value of a lateral width of the absorbent main body.

3. The underpants-shaped absorbent article according to claim 2, wherein
in the lateral direction,
a distance between an outermost end of the absorbent main body and an innermost end of the waist member
is less than 1/2 of
a distance between an outermost end of the waist member and the innermost end of the waist member.

4. The underpants-shaped absorbent article according to claim 1, wherein
in an unfolded state,
a length of a portion that extends along the second region (72) and that is located on a front side with respect to a central position of the underpants-shaped absorbent article in the longitudinal direction
is longer than
a length of a portion that extends along the second region and that is located on a back side with respect to the central position.

5. The underpants-shaped absorbent article according to claim4, wherein
in an unfolded state,
a length of a portion that extends along the first region (71) and that is located on a back side with respect to a central position of the underpants-shaped absorbent article in the longitudinal direction
is longer than
a length of a portion that extends along the first region and that is located on a front side with respect to the central position.

6. The underpants-shaped absorbent article according to claim 1, wherein
the front waist portion has a front skin-side sheet (21) and a front non-skin-side sheet (32),
the back waist portion has a back skin-side sheet (21) and a back non-skin-side sheet (42),
the front skin-side sheet and the back skin-side sheet are formed of an integral sheet member that is continuous in the longitudinal direction in an unfolded state,
the front non-skin-side sheet and the back non-skin-side sheet are formed of different sheet members that are non-continuous in the longitudinal direction in the unfolded state,
the waist member is joined to a non-skin side of the absorbent main body,
the different sheet members that constitute the front non-skin-side sheet and the back non-skin-side sheet are hydrophilic nonwoven fabrics, and
the integral sheet member that constitutes the front skin-side sheet and the back skin-side sheet is a hydrophobic nonwoven fabric having a lower hydrophilicity than the hydrophilic nonwoven fabric.

7. The underpants-shaped absorbent article according to claim 6, wherein
a minimum value of a lateral width of the waist member is larger than a minimum value of a lateral width of the absorbent main body,
the leg opening (LH) is formed of the waist member and is located below a lower end of the side joining portion,
the leg opening has a first region(71f) and a second region (71r),
the first region including the front non-skin-side sheet and the back non-skin-side sheet,
the second region not including the front non-skin-side sheet and the back non-skin-side sheet, and being constituted by the front skin-side sheet and the back skin-side sheet, and
a portion of the first region farthest on the non-skin side is a hydrophilic region, and
a portion of the second region farthest on the non-skin side is a hydrophobic region.

8. The underpants-shaped absorbent article according to claim 7, wherein
in the second region,
a sheet member arranged on a non-skin side with respect to the absorbent main body is only one sheet of the hydrophobic nonwoven fabric.

9. The underpants-shaped absorbent article according to claim 7, wherein
a minimum value of a lateral width of the waist member is smaller than a minimum value of a lateral width of the absorbent main body,
the leg opening has a first region and a second region on and below a lower end of the side joining portion,
the first region being constituted by the waist member,
the second region being constituted by the absorbent main body,
a portion of the first region farthest on the non-skin side is a hydrophilic region, and
a portion of the second region farthest on the non-skin side is a hydrophobic region.

10. The underpants-shaped absorbent article according to any one of claims 7 to 9, wherein
in an unfolded state,
a length of a portion that extends along the second region and that is located on a front side with respect to a central position of the underpants-shaped absorbent article in the longitudinal direction
is longer than
a length of a portion that extends along the second region and that is located on a back side with respect to the central position.

11. The underpants-shaped absorbent article according to any one of claims 7 to 10, wherein
in an unfolded state,
a length of a portion that extends along the first region and that is located on a back side with respect to a central position of the underpants-shaped absorbent article in the longitudinal direction
is longer than
a length of a portion that extends along the first region and that is located on a front side with respect to the central position.

12. The underpants-shaped absorbent article according to any one of claims 1 to 11, wherein
a farthest-on-non-skin-side layer of each of the front waist portion and the back waist portion is formed of one sheet of hydrophilic nonwoven fabric.

13. The underpants-shaped absorbent article according to claims 1 to 12, wherein
the back waist portion has a buttocks cover (40b) below lower ends of the side joining portions,
the buttocks cover having a lateral width that is narrowed from an upper side to a lower side in the vertical direction, and
an elastic member (47) that stretches and contracts along an end edge portion of the buttocks cover is provided.

## Patentansprüche

1. Unterhosen-förmiger absorbierender Artikel, der eine vertikale Richtung, eine laterale Richtung und eine Vorn-Hinten-Richtung aufweist, die einander schneiden,
wobei der Unterhosen-förmige absorbierende Artikel Folgendes umfasst:
einen flüssigkeitsabsorbierenden absorbierenden Hauptkörper (10);
ein Taillenelement (20), in dem ein vorderer Taillenteil (30) und ein hinterer Taillenteil (40) ringförmig durch ein Paar von Seitenverbindungsteilen, die an zwei Endteilen in der lateralen Richtung bereitgestellt sind, verbunden sind; und
ein Paar von Beinöffnungen (LH, LH), durch die Beine des Trägers hindurchgehen,
in dem Taillenelement, zumindest in einem Teil eines Bereichs, der mit dem Paar von Seitenverbindungsteilen in Bezug auf die vertikale Richtung überlappt, einen hydrophilen Bereich, der am weitesten auf einer Nicht-Hautseite bereitgestellt ist,
in Bereichen, die sich entlang der Beinöffnungen erstrecken, zumindest in einem Teil, der sich an einem untersten Ende befindet, einen hydrophoben Bereich, der am weitesten auf der Nicht-Hautseite bereitgestellt ist,
wobei der hydrophobe Bereich eine geringere Hydrophilie aufweist als der hydrophile Bereich, wobei:
in einem entfalteten Zustand
der vordere Taillenteil mit einer Seite des absorbierenden Hauptkörpers in einer Längsrichtung verbunden ist und
der hintere Taillenteil mit einer anderen Seite in der Längsrichtung verbunden ist,
ein hydrophiler Vliesstoff am weitesten auf der Nicht-Hautseite in zumindest einem des vorderen Taillenteils und des hinteren Taillenteils bereitgestellt ist und
ein hydrophober Vliesstoff am weitesten auf einer Nicht-Hautseite in dem absorbierenden Hauptkörper bereitgestellt ist,
wobei der hydrophobe Vliesstoff eine geringere Hydrophilie aufweist als der hydrophile Vliesstoff, und **dadurch gekennzeichnet ist, dass**:
die Beinöffnung einen ersten Bereich (71) und einen zweiten Bereich (72) an oder unter einem unteren Ende des Seitenverbindungsteils aufweist,
wobei der erste Bereich durch das Taillenelement gebildet ist,
wobei der zweite Bereich durch den absorbierenden Hauptkörper gebildet ist,
ein Teil des ersten Bereichs am weitesten auf der Nicht-Hautseite ein hydrophiler Bereich ist und
ein Teil des zweiten Bereichs am weitesten auf der Nicht-Hautseite ein hydrophober Bereich ist.

2. Unterhosen-förmiger absorbierender Artikel nach Anspruch 1, wobei
in einem entfalteten Zustand
das Taillenelement integral derart ausgebildet ist, dass der vordere Taillenteil und der hintere Taillenteil in einer Längsrichtung kontinuierlich sind,
das Taillenelement mit einer Nicht-Hautseite des absorbierenden Hauptkörpers verbunden ist,
ein hydrophiler Vliesstoff am weitesten auf einer Nicht-Hautseite des Taillenelements bereitgestellt ist,
ein hydrophober Vliesstoff am weitesten auf einer Nicht-Hautseite des absorbierenden Hauptkörpers bereitgestellt ist,
wobei der hydrophobe Vliesstoff eine geringere Hydrophilie aufweist als der hydrophile Vliesstoff, und
ein minimaler Wert einer lateralen Breite des Taillenelements kleiner ist als ein minimaler Wert einer lateralen Breite des absorbierenden Hauptkörpers.

3. Unterhosen-förmiger absorbierender Artikel nach Anspruch 2, wobei
in der lateralen Richtung
ein Abstand zwischen einem äußersten Ende des absorbierenden Hauptkörper und einem innersten Ende des Taillenelements
kleiner ist als 1/2
eines Abstands zwischen einem äußersten Ende des Taillenelements und dem innersten Ende des Taillenelements.

4. Unterhosen-förmiger absorbierender Artikel nach Anspruch 1, wobei
in einem entfalteten Zustand
eine Länge eines Teils, der sich entlang des zweiten Bereichs (72) erstreckt und sich auf einer vorderen Seite in Bezug auf eine mittlere Position des Unterhosenförmigen absorbierenden Artikels in der Längsrichtung befindet,
länger ist als
eine Länge eines Teils, der sich entlang des zweiten Bereichs erstreckt und sich auf einer hinteren Seite in Bezug auf eine mittlere Position befindet.

5. Unterhosen-förmiger absorbierender Artikel nach Anspruch 4, wobei
in einem entfalteten Zustand
eine Länge eines Teils, der sich entlang des ersten Bereichs (71) erstreckt und sich auf einer hinteren Seite in Bezug auf eine mittlere Position des Unterhosenförmigen absorbierenden Artikels in der Längsrichtung befindet,
länger ist als
eine Länge eines Teils, der sich entlang des ersten Bereichs erstreckt und sich auf einer vorderen Seite in Bezug auf eine mittlere Position befindet.

6. Unterhosen-förmiger absorbierender Artikel nach Anspruch 1, wobei
der vordere Taillenteil eine vordere Hautseitenlage (21) und eine vordere Nicht-Hautseitenlage (32) aufweist,
der hintere Taillenteil eine hintere Hautseitenlage (21) und eine hintere Nicht-Hautseitenlage (42) aufweist,
die vordere Hautseitenlage und die hintere Hautseitenlage aus einem integralen Lagenelement ausgebildet sind, das in der Längsrichtung in einem entfalteten Zustand kontinuierlich ist,
die vordere Nicht-Hautseitenlage und die hintere Nicht-Hautseitenlage aus verschiedenen Lagenelementen ausgebildet sind, die in der Längsrichtung in dem entfalteten Zustand nicht kontinuierlich sind,
das Taillenelement mit einer Nicht-Hautseite des absorbierenden Hauptkörpers verbunden ist,
die verschiedenen Lagenelemente, die die vordere Nicht-Hautseitenlage und die hintere Nicht-Hautseitenlage bilden, hydrophile Vliesstoffe sind und
das integrale Lagenelement, das die vordere Hautseitenlage und die hintere Hautseitenlage bildet, ein hydrophober Vliesstoff ist, der eine geringere Hydrophilie aufweist als der hydrophile Vliesstoff.

7. Unterhosen-förmiger absorbierender Artikel nach Anspruch 6, wobei
ein minimaler Wert einer lateralen Breite des Taillenelements größer ist als ein minimaler Wert einer lateralen Breite des absorbierenden Hauptkörpers,
die Beinöffnung (LH) aus dem Taillenelement ausgebildet ist und sich unter einem unteren Ende des Seitenverbindungsteils befindet,
die Beinöffnung einen ersten Bereich (71f) und einen zweiten Bereich (71r) aufweist,
wobei der erste Bereich die vordere Nicht-Hautseitenlage und die hintere Nicht-Hautseitenlage einschließt,
wobei der zweite Bereich die vordere Nicht-Hautseitenlage und die hintere Nicht-Hautseitenlage nicht einschließt und aus der vorderen Hautseitenlage und der hinteren Hautseitenlage gebildet ist, und
ein Teil des ersten Bereichs am weitesten auf der Nicht-Hautseite ein hydrophiler Bereich ist und
ein Teil des zweiten Bereichs am weitesten auf der Nicht-Hautseite ein hydrophober Bereich ist.

8. Unterhosen-förmiger absorbierender Artikel nach Anspruch 7, wobei
in dem zweiten Bereich
ein Lagenelement, das auf einer Nicht-Hautseite in Bezug auf den absorbierenden Hauptkörper angeordnet ist, nur eine Lage des hydrophoben Vliesstoffs ist.

9. Unterhosen-förmiger absorbierender Artikel nach Anspruch 7, wobei
ein minimaler Wert einer lateralen Breite des Taillenelements kleiner ist als ein minimaler Wert einer lateralen Breite des absorbierenden Hauptkörpers,
die Beinöffnung einen ersten Bereich und einen zweiten Bereich an und unter einem unteren Ende des Seitenverbindungsteils aufweist,
wobei der erste Bereich durch das Taillenelement gebildet ist,
wobei der zweite Bereich durch den absorbierenden Hauptkörper gebildet ist,
ein Teil des ersten Bereichs am weitesten auf der Nicht-Hautseite ein hydrophiler Bereich ist und
ein Teil des zweiten Bereichs am weitesten auf der Nicht-Hautseite ein hydrophober Bereich ist.

10. Unterhosen-förmiger absorbierender Artikel nach einem der Ansprüche 7 bis 9, wobei
in einem entfalteten Zustand
eine Länge eines Teils, der sich entlang des zweiten Bereichs erstreckt und sich auf einer vorderen Seite in Bezug auf eine mittlere Position des Unterhosenförmigen absorbierenden Artikels in der Längsrichtung befindet,
länger ist als
eine Länge eines Teils, der sich entlang des zweiten Bereichs erstreckt und sich auf einer hinteren Seite in Bezug auf die mittlere Position befindet.

11. Unterhosen-förmiger absorbierender Artikel nach einem der Ansprüche 7 bis 10, wobei
in einem entfalteten Zustand
eine Länge eines Teils, der sich entlang des ersten Bereichs erstreckt und sich auf einer hinteren Seite in Bezug auf eine mittlere Position des Unterhosenförmigen absorbierenden Artikels in der Längsrichtung befindet,
länger ist als
eine Länge eines Teils, der sich entlang des ersten Bereichs erstreckt und sich auf einer vorderen Seite in Bezug auf die mittlere Position befindet.

12. Unterhosen-förmiger absorbierender Artikel nach einem der Ansprüche 1 bis 11, wobei
eine Schicht am weitesten auf der Nicht-Hautseite von jedem des vorderen Taillenteils und des hinteren Taillenteils aus einer Lage von hydrophilem Vliesstoff ausgebildet ist.

13. Unterhosen-förmiger absorbierender Artikel nach den Ansprüchen 1 bis 12, wobei
der hintere Taillenteil eine Gesäßabdeckung (40b) unter unteren Enden der Seitenverbindungsteile aufweist,
wobei die Gesäßabdeckung eine laterale Breite aufweist, die sich von einer oberen Seite zu einer unteren Seite in der vertikalen Richtung verengt, und
ein elastisches Element (47), das sich entlang eines Endrandteils der Gesäßabdeckung dehnt und zusammenzieht, bereitgestellt ist.

## Revendications

1. Article absorbant en forme de culotte ayant une direction verticale, une direction latérale et une direction allant d'avant en arrière qui se croisent les unes les autres,
l'article absorbant en forme de culotte comportant :
un corps principal absorbant qui absorbe les liquides (10) ;
un élément au niveau de la taille (20) dans lequel une partie avant au niveau de la taille (30) et une partie arrière au niveau de la taille (40) sont assemblées de manière annulaire par une paire de parties d'assemblage latérales qui sont mises en œuvre au niveau de deux parties d'extrémité dans la direction latérale ; et
une paire d'ouvertures pour jambe (LH, LH) au travers desquelles les jambes de l'utilisateur passent,
dans l'élément au niveau de la taille, au moins dans une partie d'une région qui chevauche la paire de parties d'assemblage latérales par rapport à la direction verticale, une région hydrophile étant mise en œuvre le plus loin sur un côté non orienté vers la peau,
dans des régions s'étendant le long des ouvertures pour jambe, au moins dans une partie qui est située au niveau de l'extrémité se trouvant le plus en bas, une région hydrophobe étant mise en œuvre le plus loin sur le côté non orienté vers la peau,
la région hydrophobe ayant une hydrophilie inférieure par rapport à celle de la région hydrophile, dans lequel ;
dans un état déplié,
la partie avant au niveau de la taille est assemblée sur un côté du corps principal absorbant dans une direction longitudinale, et
la partie arrière au niveau de la taille est assemblée sur un autre côté dans la direction longitudinale,
un tissu non tissé hydrophile est mis en œuvre le plus loin sur le côté non orienté vers la peau dans au moins l'une parmi la partie avant au niveau de la taille et la partie arrière au niveau de la taille, et
un tissu non tissé hydrophobe est mis en œuvre le plus loin sur un côté non orienté vers la peau dans le corps principal absorbant,
le tissu non tissé hydrophobe ayant une hydrophilie inférieure par rapport à celle du tissu non tissé hydrophile, et **caractérisé en ce que** ;
l'ouverture pour jambe a une première région (71) et une deuxième région (72) sur et sous une extrémité inférieure de la partie d'assemblage latérale,
la première région étant constituée par l'élément au niveau de la taille,
la deuxième région étant constituée par le corps principal absorbant,
une partie de la première région se trouvant le plus loin sur le côté non orienté vers la peau est une région hydrophile, et
une partie de la deuxième région se trouvant le plus loin sur le côté non orienté vers la peau est une région hydrophobe.

2. Article absorbant en forme de culotte selon la revendication 1, dans lequel
dans un état déplié,
l'élément au niveau de la taille est constitué d'une seule pièce de telle sorte que la partie avant au niveau de la taille et la partie arrière au niveau de la taille sont continues dans une direction longitudinale,
l'élément au niveau de la taille est assemblé sur un côté non orienté vers la peau du corps principal absorbant,
un tissu non tissé hydrophile est mis en œuvre le plus loin sur un côté non orienté vers la peau de l'élément au niveau de la taille,
un tissu non tissé hydrophobe est mis en œuvre le plus loin sur un côté non orienté vers la peau du corps principal absorbant,
le tissu non tissé hydrophobe ayant une hydrophilie inférieure par rapport à celle du tissu non tissé hydrophile, et
une valeur minimale d'une largeur latérale de l'élément au niveau de la taille est inférieure à une valeur minimale d'une largeur latérale du corps principal absorbant.

3. Article absorbant en forme de culotte selon la revendication 2, dans lequel
dans la direction latérale,
une distance entre une extrémité se trouvant le plus à l'extérieur du corps principal absorbant et une extrémité se trouvant le plus à l'intérieur de l'élément au niveau de la taille
fait moins de la moitié (1/2) si l'on compare à
une distance entre une extrémité se trouvant le plus à l'extérieur de l'élément au niveau de la taille et l'extrémité se trouvant le plus à l'intérieur de l'élément au niveau de la taille.

4. Article absorbant en forme de culotte selon la revendication 1, dans lequel
dans un état déplié,
une longueur d'une partie qui s'étend le long de la deuxième région (72) et qui est située sur un côté avant par rapport à une position centrale de l'article absorbant en forme de culotte dans la direction longitudinale
est plus longue si l'on compare à
une longueur d'une partie qui s'étend le long de la deuxième région et qui est située sur un côté arrière par rapport à la position centrale.

5. Article absorbant en forme de culotte selon la revendication 4, dans lequel
dans un état déplié,
une longueur d'une partie qui s'étend le long de la première région (71) et qui est située sur un côté arrière par rapport à une position centrale de l'article absorbant en forme de culotte dans la direction longitudinale
est plus longue si l'on compare à
une longueur d'une partie qui s'étend le long de la première région et qui est située sur un côté avant par rapport à la position centrale.

6. Article absorbant en forme de culotte selon la revendication 1, dans lequel
la partie avant au niveau de la taille a une feuille avant côté orienté vers la peau (21) et une feuille avant côté non orienté vers la peau (32),
la partie arrière au niveau de la taille a une feuille arrière côté orienté vers la peau (21) et une feuille arrière côté non orienté vers la peau (42),
la feuille avant côté orienté vers la peau et la feuille arrière côté orienté vers la peau sont formées à partir d'un élément formant feuille intégré qui est continu dans la direction longitudinale dans un état déplié,
la feuille avant côté non orienté vers la peau et la feuille arrière côté non orienté vers la peau sont formées à partir de différents éléments formant feuille qui sont non continus dans la direction longitudinale dans l'état déplié,
l'élément au niveau de la taille est assemblé sur un côté non orienté vers la peau du corps principal absorbant,
les différents éléments formant feuille qui constituent la feuille avant côté non orienté vers la peau et la feuille arrière côté non orienté vers la peau sont des tissus non tissés hydrophiles, et
l'élément formant feuille intégré qui constitue la feuille avant côté orienté vers la peau et la feuille arrière côté orienté vers la peau est un tissu non tissé hydrophobe ayant une hydrophilie inférieure par rapport à celle du tissu non tissé hydrophile.

7. Article absorbant en forme de culotte selon la revendication 6, dans lequel
une valeur minimale d'une largeur latérale de l'élément au niveau de la taille est supérieure à une valeur minimale d'une largeur latérale du corps principal absorbant,
l'ouverture pour jambe (LH) est formée à partir de l'élément au niveau de la taille et est située sous une extrémité inférieure de la partie d'assemblage latérale,
l'ouverture pour jambe a une première région (71f) et une deuxième région (71r),
la première région comprenant la feuille avant côté non orienté vers la peau et la feuille arrière côté non orienté vers la peau,
la deuxième région ne comprenant pas la feuille avant côté non orienté vers la peau et la feuille arrière côté non orienté vers la peau, et étant constituée par la feuille avant côté orienté vers la peau et la feuille arrière côté orienté vers la peau, et
une partie de la première région se trouvant le plus loin sur le côté non orienté vers la peau est une région hydrophile, et
une partie de la deuxième région se trouvant le plus loin sur le côté non orienté vers la peau est une région hydrophobe.

8. Article absorbant en forme de culotte selon la revendication 7, dans lequel
dans la deuxième région,
un élément formant feuille agencé sur un côté non orienté vers la peau par rapport au corps principal absorbant est une seule feuille du tissu non tissé hydrophobe.

9. Article absorbant en forme de culotte selon la revendication 7, dans lequel
une valeur minimale d'une largeur latérale de l'élément au niveau de la taille est inférieure à une valeur minimale d'une largeur latérale du corps principal absorbant,
l'ouverture pour jambe a une première région et une deuxième région sur et sous une extrémité inférieure de la partie d'assemblage latérale,
la première région étant constituée par l'élément au niveau de la taille,
la deuxième région étant constituée par le corps principal absorbant,
une partie de la première région se trouvant le plus loin sur le côté non orienté vers la peau est une région hydrophile, et
une partie de la deuxième région se trouvant le plus loin sur le côté non orienté vers la peau est une région hydrophobe.

10. Article absorbant en forme de culotte selon l'une quelconque des revendications 7 à 9, dans lequel
dans un état déplié,
une longueur d'une partie qui s'étend le long de la deuxième région et qui est située sur un côté avant par rapport à une position centrale de l'article absorbant en forme de culotte dans la direction longitudinale
est plus longue si l'on compare à
une longueur d'une partie qui s'étend le long de la deuxième région et qui est située sur un côté arrière par rapport à la position centrale.

11. Article absorbant en forme de culotte selon l'une quelconque des revendications 7 à 10, dans lequel
dans un état déplié,
une longueur d'une partie qui s'étend le long de la première région et qui est située sur un côté arrière par rapport à une position centrale de l'article absorbant en forme de culotte dans la direction longitudinale
est plus longue si l'on compare à
une longueur d'une partie qui s'étend le long de la première région et qui est située sur un côté avant par rapport à la position centrale.

12. Article absorbant en forme de culotte selon l'une quelconque des revendications 1 à 11, dans lequel
une couche située le plus loin sur le côté non orienté vers la peau de chacune parmi la partie avant au niveau de la taille et la partie arrière au niveau de la taille est formée à partir d'une feuille de tissu non tissé hydrophile.

13. Article absorbant en forme de culotte selon l'une quelconque des revendications 1 à 12, dans lequel
la partie arrière au niveau de la taille a un couvre-fesses (40b) sous les extrémités inférieures des parties d'assemblage latérales,
le couvre-fesses ayant une largeur latérale qui va en se rétrécissant depuis un côté supérieur jusqu'à un côté inférieur dans la direction verticale, et
un élément élastique (47) qui s'étire et se contracte le long d'une partie de bord d'extrémité du couvre-fesses est mis en œuvre.
